# EUROPÄISCHE PATENTSCHRIFT

(11) **EP 4 007 641 B1**
(45) Veröffentlichungstag und Bekanntmachung des Hinweises auf die Patenterteilung: **06.08.2025**
(21) Anmeldenummer: 20731844.5
(22) Anmeldetag: 08.06.2020
(51) Int. Cl.: A61Q 5/06, A61Q 5/10, A61K 8/58, A61K 8/64, A61K 8/898

(54) **VERFAHREN ZUR BEHANDLUNG VON KERATINMATERIAL, UMFASSEND DIE ANWENDUNG EINES ORGANISCHEN C1-C6-ALKOXY-SILANS UND EINER AMINOSÄURE UND/ODER EINES AMINOSÄUREDERIVATS**
METHOD FOR TREATING KERATINOUS MATERIAL, COMPRISING THE USE OF AN ORGANIC C1-C6 ALKOXY SILANE AND AN AMINO ACID AND/OR AN AMINO ACID DERIVATIVE
PROCEDE DE TRAITEMENT DE MATIERES KERATINIQUES, COMPRENANT L'UTILISATION D'UN ALCOXYSILANE ORGANIQUE EN C1-C6 ET D'UN ACIDE AMINE ET/OU D'UN DERIVE D'ACIDE AMINE

(30) Priorität: 01.08.2019 DE 102019211510
(43) Veröffentlichungstag der Anmeldung: 08.06.2022
(73) Patentinhaber: Henkel AG & Co. KGaA, 40589 Düsseldorf (DE)
(72) Erfinder: NOWOTTNY, Marc, 41069 Mönchengladbach (DE); SCHOEPGENS, Juergen, 41366 Schwalmtal (DE); MATHIASZYK, Carsten, 45277 Essen (DE); JAISER, Phillip, 40764 Langenfeld (DE); SCHUMACHER, Ulrike, 40589 Düsseldorf (DE); KRIENER, Caroline, 40229 Düsseldorf (DE); KOLONKO, Claudia, 42857 Remscheid (DE); LECHNER, Torsten, 40764 Langenfeld (DE); WESER, Gabriele, 41472 Neuss (DE); HODES, Jing, 58093 Hagen (DE)
(86) Internationale Anmeldenummer: PCT/EP2020/065776
(87) Internationale Veröffentlichungsnummer: WO 2021/018443

(56) Entgegenhaltungen:
- WO-A1-2018/115059
- WO-A1-2020/089366
- WO-A2-2013/087319

## Beschreibung

Die vorliegende Anmeldung liegt auf dem Gebiet der Kosmetik und betrifft ein Verfahren zur Behandlung von keratinischem Material, insbesondere menschlichen Haaren, welches die Anwendung von zwei Zusammsetzungen (A) und (B) umfasst. Bei der Zusammensetzung (A) handelt es sich um eine Zubereitung, die mindestens ein organisches C₁-C₆-Alkoxysilan enthält, das ausgewählt ist aus der Gruppe aus (3-Aminopropyl)triethoxysilan, (3-Aminopropyl)trimethoxysilan, (2-Aminoethyl)triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxy-silan, (2-Dimethylaminoethyl)trimethoxysilan und/oder deren Kondensationsprodukten, und mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten, und die Zusammensetzung (B) besitzt einen pH-Wert von 8,0 bis 10,5 und beinhaltet mindestens eine Verbindung (B1), die ausgewählt ist aus der Gruppe aus Aminosäuren und Proteinhydrolysaten. Hierbei sind die beiden Zusammensetzungen (A) und (B) dadurch gekennzeichnet, dass ihr Polymergehalt jeweils auf einen bestimmten Maximalgehalt beschränkt ist

Ein zweiter Gegenstand der vorliegenden Erfindung ist eine Mehrkomponenten-Verpackungseinheit (Kit-of-parts) zum Färben von keratinischem Material, welche getrennt konfektioniert in zwei Verpackungseinheiten die zwei zuvor beschriebenen Zusammensetzungen (A) und (B) umfasst

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

WO2020/089366A1 besitzt einen Prioritätstag, der vor dem Prioritätstag der vorliegenden Erfindung liegt, wurde aber nach letzterem veröffentlicht. Dieses Dokument stellt damit Stand der Technik gemäß Art. 54(3) EPÜ dar. Gegenstand der WO 2020/089366 A1 sind kosmetische Mittel zur Behandlung von keratinischem Material, umfassend mindestens eine organische Siliciumverbindung (a) und eine organische und/oder anorganische Säure mit einem pKs-Wert von -6 bis 5 (b).

WO 2013/087319 A2 beschreibt Färbemittel, die eine verbesserte Färbung bewirken sollen. Zu diesem Zweck schlägt WO 2013/087319 A2 die Verwendung einer Wirkstoffkombination aus mindestens einem wasserlöslichen Polymer mit funktioneller Alkoxy-Silan-Einheit (a) und mindestens einer polaren Alkoxysilanverbindung (b) einer Formel (SI) vor.

WO 2018/115059 A1 betrifft ein Verfahren zum Färben von Keratinfasern, umfassend die Anwendung eines Mittels (A), welches zwei voneinander verschiedene organische Silane enthält, und die Anwendung eines Mittels (B) mit anionischem Direktziehendem Farbstoff und Tensid, wobei das Mittel (B) nach dem Mittel (A) angewendet wird.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

Wenn diese Alkoxy-Silane bzw. ihre Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern und resultiert in überraschend waschbeständigen Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach kurzen Anwendungszeiträumen von nur wenigen Minuten gute Färbeergebnisse erzielt werden.

In den Dokumenten des Standes der Technik, wie beispielsweise in EP 2168633 B1, werden zur Erhöhung der Echtheitseigenschaften oft filmbildende Polymere im Haarfärbeverfahren eingesetzt. Wenn der Einsatz dieser Polymere auch gewisse Vorteile im Hinblick auf die Erhöhung der Waschechtheiten bringt, so sind mit ihrer Anwendung doch auch diverse Nachteile verbunden. Die im Färbeverfahren der EP 2168633 B1 eingesetzten Polymere lagern sich aufgrund ihrer filmbildenden Eigenschaften auf dem Keratinmaterial ab, so dass neben den organischen Silicium-Verbindungen eine zweite Substanzklasse anwendet wird, die zu einem Coating oder einem Überzug auf dem Keratinmaterial führt. Diese verstärkte Filmbildung bzw. die Ausbildung sehr dicker Filme kann in nachteiliger Weise das Haar beschweren, so dass der Anwender schlimmstenfalls eine zusammengefallene Frisur, wenig Volumen und ein unangenehmes Griffgefühl auf seinem Haar wahrnimmt. Zudem hat sich herausgestellt, dass die Anwesenheit des Polymeres bei Wiederholung der Färbung zu Problemen führen kann. Wo bei der Erstanwendung dieses Färbesystems noch sehr zufriedenstellende Farbintensitäten erzielt werden konnten, stellte sich bei der Folgeanwendung heraus, dass anscheinend noch verbleibende Polymer-Rückstände die Farbintensität der zweiten Färbung negativ beeinflussten. Aus diesem Grund besteht nach wie vor großer Bedarf an Pigment-Färbesystemen, die auch ohne Einsatz von Polymern zu intensiven und waschbeständigen Färbungen führen und die vor allem auch bei wiederholter Anwendung nicht mit einer Verminderung der Farbintensität verbunden sind.

Es war daher die Aufgabe der vorliegenden Anmeldung, ein Verfahren zum Behandeln von keratinischem Material aufzufinden, das insbesondere auch in der Färbung von Haaren eingesetzt werden kann und hierbei Verbesserungen im Hinblick auf die Farbintensität und die Waschechtheit zeigt. Bei wiederholter Anwendung sollte die Färbeleistung noch genau so hoch wie bei der Erstanwendung sein. Die Farbintensitäten und Waschechtheiten sollten im Vergleich zu den Färbungen, die bislang mit den aus dem Stand der Technik bekannten Formulierungen erzielt werden können, verbessert sein. Aufgrund der zuvor beschriebenen Nachteile sollte zur Lösung dieser Aufgabe auf die Anwesenheit von Polymeren verzichtet werden.

Überraschenderweise hat sich herausgestellt, dass diese Aufgabe in vollem Umfang gelöst werden kann, wenn das Keratinmaterial in einem Verfahren behandelt wird, bei dem zwei Zusammensetzungen (A) und (B) auf dem Keratinmaterial angewendet werden. Hierbei enthält die erste Zusammensetzung (A) mindestens ein organisches C₁-C₆-Alkoxy-Silan, das ausgewählt ist aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxy-silan, (2-Dimethylaminoethyl)-trimethoxysilan und/oder deren Kondensationsprodukten, und mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten, und die zweite Zusammensetzung (B) ist gekennzeichnet durch ihren pH-Wert von 8,0 bis 10,5 und ihren Gehalt an mindestens einer Verbindung, die ausgewählt ist aus der Gruppe der Aminosäuren und der Proteinhydrolysate. Zur Erzielung entsprechend guter Färbeleistungen auch bei wiederholter Anwendung war weder in der Zusammensetzung (A) noch in der Zusammensetzung (B) die Anwesenheit eines Polymers erforderlich.

Ein erster Gegenstand der vorliegenden Erfindung ist ein Verfahren zum Behandeln von keratinischem Material, insbesondere menschlichen Haaren, bei dem auf dem keratinischen Material angewendet werden:
- eine erste Zusammensetzung (A), die enthält:
   (A1) mindestens ein organisches C₁-C₆-Alkoxy-Silan, das ausgewählt ist aus der Gruppe aus (3-Aminopropyl)triethoxysilan, (3-Aminopropyl)-trimethoxysilan, (2-Aminoethyl)triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxy-silan, (2-Dimethylaminoethyl)trimethoxysilan und/oder deren Kondensationsprodukten, und
   (A12) mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten, und
- eine zweite Zusammensetzung (B), die einen pH-Wert von 8,0 bis 10,5 besitzt und die enthält
   (B1) mindestens eine Verbindung, die ausgewählt ist aus der Gruppe aus Aminosäuren und Proteinhydrolysaten,
wobei
- der Gesamtgehalt aller in der Zusammensetzung (A) enthaltenen Polymere - bezogen auf das Gesamtgewicht der Zusammensetzung (A) - unterhalb von 0,1 Gew.-% liegt, und
- der Gesamtgehalt aller in der Zusammensetzung (B) enthaltenen Polymere - bezogen auf das Gesamtgewicht der Zusammensetzung (B) - unterhalb von 0,1 Gew.-% liegt.

Wurde die Zusammensetzung (A) im Rahmen eines Färbeverfahrens auf dem Keratinmaterial appliziert, so konnte eine Erhöhung der Farbintensität vor allem dann festgestellt werden, wenn die Zusammensetzung (B) in Form eines Nachbehandlungsmittels nach Anwendung der Zusammensetzung (A) auf dem Keratinmaterial appliziert wurde. Neben der Verstärkung der Farbintensität konnte in diesem Zusammenhang überraschenderweise auch eine Verbesserung der Waschechtheit beobachtet werden. Diese Effekte konnte auch ohne die Anwesenheit eines Polymers in den Zusammensetzungen (A) und (B) erzielt werden. Insbesondere bei Wiederholung des Färbeverfahrens wurde bei der Folgeanwendung keine Abschwächung der Farbintensität beobachtet.

### Behandlung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter Mitteln zur Behandlung von keratinischem Material werden beispielsweise Mittel zur Färbung des Keratinmaterials, Mittel zur Umformung oder Formgebung von keratinischem Material, insbesondere keratinischen Fasern, oder auch Mittel zur Konditionierung bzw. zur Pflege des keratinischen Materials verstanden. Besonders gute Eignung zeige die über das erfindungsgemäße Verfahren hergestellten Mittel zur Färbung von keratinischem Material, insbesondere zur Färbung von keratinischen Fasern, bei denen es sich besonders bevorzugt um menschliche Haare handelt.

Der Begriff "Mittel zur Färbung" wird im Rahmen dieser Erfindung für eine durch Einsatz von farbgebenden Verbindungen, wie beispielsweise thermochromen und photochromen Farbstoffen, Pigmenten, Mica, direktziehenden Farbstoffen und/oder Oxidationsfarbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Polymerisierung des oder der organischen Alkoxy-Silane, und durch die Wechselwirkung von farbgebender Verbindung und organischer Siliciumverbindung und optional weiteren Bestandteilen, wie beispielsweise einem filmbildenden, Polymer.

### Organische C₁-C₆-Alkoxy-Silane (A1) und/oder deren Kondensationsprodukte in der Zusammensetzung (A)

Die Zusammensetzung (A) ist dadurch gekennzeichnet, dass sie
(A11) mindestens ein organisches C₁-C₆-Alkoxy-Silan, das ausgewählt ist aus der Gruppe aus (3-Aminopropyl)triethoxysilan, (3-Aminopropyl)trimethoxysilan, (2-Aminoethyl)triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxy-silan, (2-Dimethylaminoethyl)trimethoxysilan und/oder deren Kondensationsprodukten, und
(A12) mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten enthält.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt.

Kennzeichnend für die erfindungsgemäßen C₁-C₆-Alkoxy-Silane ist, dass mindestens eine C₁-C₆-Alkoxygruppe direkt an ein Siliciumatom gebunden vorliegt. Die erfindungsgemäßen C₁-C₆-Alkoxy-Silane umfassen damit mindestens eine Struktureinheit R'R"R‴Si-O-(C₁-C₆-Alkyl) wobei die Reste R', R" und R‴ für die drei übrigen Bindungsvalenzen des Siliciumatoms stehen.

Das oder diese an das Siliciumatom gebundenen C₁-C₆-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren C₁-C₆-Alkoxy-Gruppe um eine Ethoxygruppe, so enthält die organische Siliciumverbindung bevorzugt eine Struktureinheit R'R"R‴Si-O-CH2-CH3. Die Reste R', R" und R‴ stellen wieder die drei übrigen freien Valenzen des Siliciumatoms dar.

Bereits der Zusatz geringer Wassermengen führt zunächst zur Hydrolyse und dann zu einer Kondensationsreaktion der organischen Alkoxy-silane untereinander. Aus diesem Grund können sowohl die organischen Alkoxy-silane (A1) als auch deren Kondensationsprodukte in der Zusammensetzung enthalten sein.

Unter einem Kondensationsprodukt wird ein Produkt verstanden, dass durch Reaktion von mindestens zwei organischen C₁-C₆-Alkoxy-Silanen unter Abspaltung von Wasser und/oder unter Abspaltung von einem C₁-C₆-Alkanol entsteht.

Die Kondensationsprodukte können beispielsweise Dimere, aber auch Trimere oder Oligomere sein, wobei die Kondensationsprodukte mit den Monomeren im Gleichgewicht stehen.

Abhängig von der eingesetzten bzw. in der Hydrolyse verbrauchten Wassermenge verschiebt sich das Gleichgewicht von monomerem C₁-C₆-Alkoxysilan zu Kondensationsprodukt.

Zur Lösung der erfindungsgemäßen Aufgabenstellung besonders gut geeignete organische Silicumverbindungen der sind
- (3-Aminopropyl)triethoxysilan 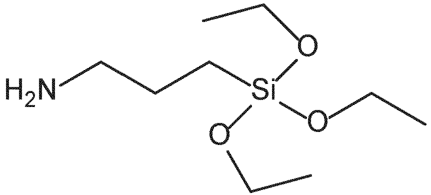
- (3-Aminopropyl)trimethoxysilan 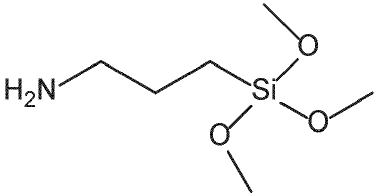
- (2-Aminoethyl)triethoxysilan 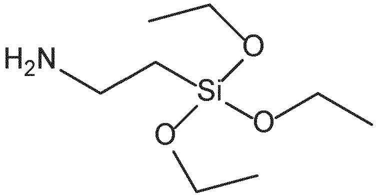
- (2-Aminoethyl)trimethoxysilan 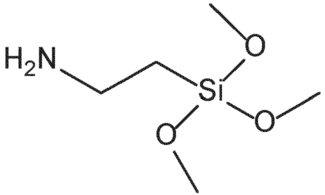
- (3-Dimethylaminopropyl)triethoxysilan 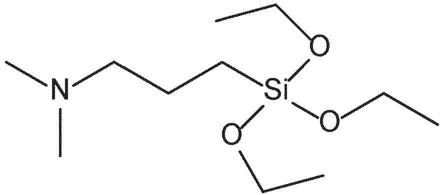
- (3-Dimethylaminopropyl)trimethoxysilan 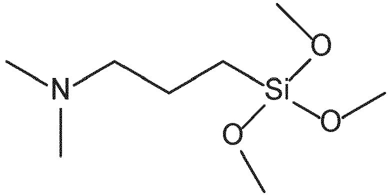
- (2-Dimethylaminoethyl)triethoxysilan. 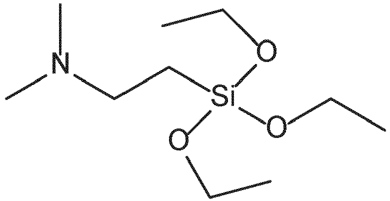
- (2-Dimethylaminoethyl)trimethoxysilan und/oder

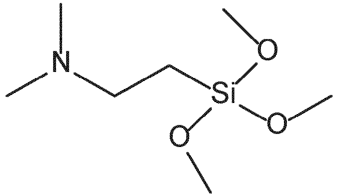

Das erfindungsgemäße Verfahren ist dadurch gekennzeichnet, dass das die erste Zusammensetzung (A) mindestens ein organisches C₁-C₆-Alkoxysilan (A11) enthält, das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten.

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Färbungen mit den besten Waschechtheiten konnten erhalten werden, wenn die Zusammensetzung (A) mindestens ein organisches C₁-C₆-Alkoxy-Silan (A12) enthält, das ausgewählt ist aus der Gruppe aus
- Methyltrimethoxysilan 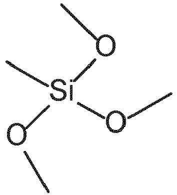
- Methyltriethoxysilan 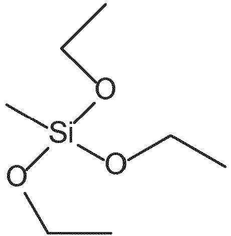
- Ethyltrimethoxysilan 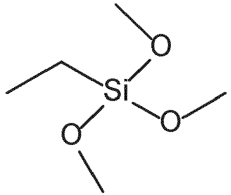
- Ethyltriethoxysilan 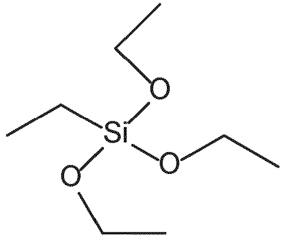
- n-Hexyltrimethoxysilan (auch bezeichnet als Hexyltrimethoxysilan) 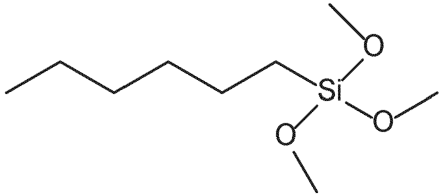
- n-Hexyltriethoxysilan (auch bezeichnet als Hexyltriethoxysilan) 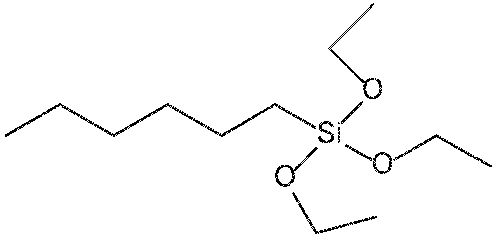
- n-Octyltrimethoxysilan (auch bezeichnet als Octyltrimethoxysilan) 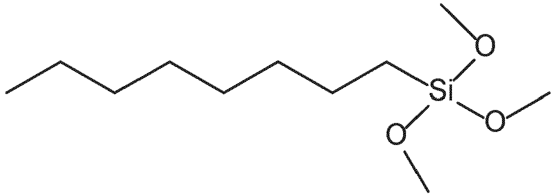
- n-Octyltriethoxysilan (auch bezeichnet als Octyltriethoxysilan) 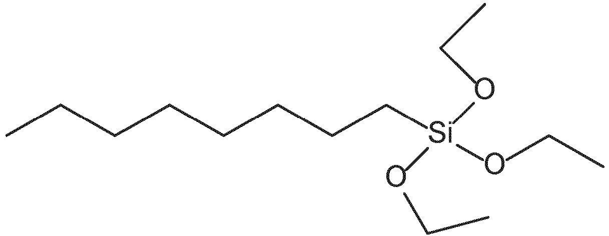
- n-Dodecyltrimethoxysilan (auch bezeichnet als Dodecyltrimethoxysilan) und/oder 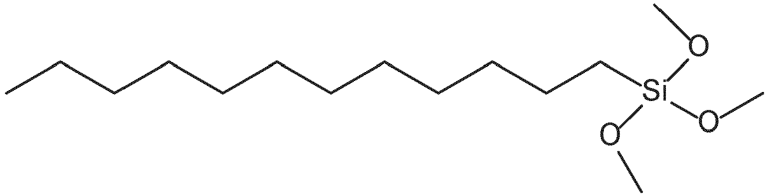
- n-Dodecyltriethoxysilan (auch bezeichnet als Dodecyltriethoxysilan).

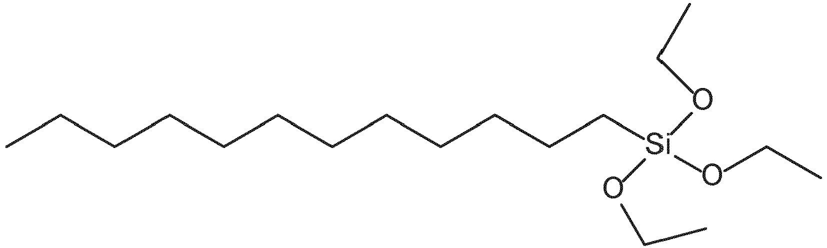

I Das erfindungsgemäße Verfahren ist dadurch gekennzeichnet, dass die erste Zusammensetzung (A) mindestens ein organisches C₁-C₆-Alkoxysilan (A12) enthält, das ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
und/oder deren Kondensationsprodukten.

Bei den entsprechenden Hydrolyse bzw. Kondensationsprodukten handelt es sich beispielsweise um die folgenden Verbindungen. Hierbei stellen die Kondensationsprodukte maximal oligomere Verbindungen, jedoch keine Polymere dar.

Hydrolyse von C₁-C₆-Alkoxy-Silan der Formel (S-I) mit Wasser (Reaktionsschema am Beispiel von 3-Aminopropyltriethoxysilan):

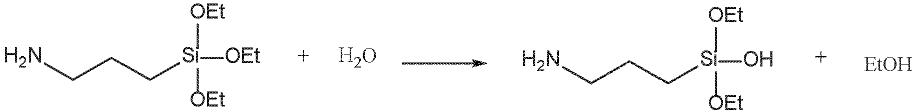

In Abhängigkeit von der eingesetzten Menge an Wasser kann die Hydrolyse-Reaktion auch mehrfach pro eingesetztem C₁-C₆-Alkoxy-Silan stattfinden: 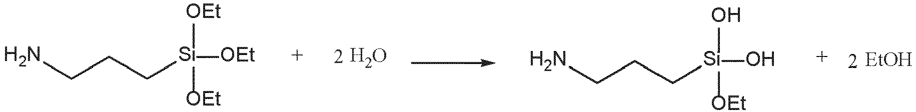 bzw.

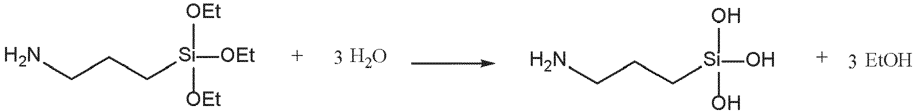

Hydrolyse von C₁-C₆-Alkoxy-Silan der Formel (S-IV) mit Wasser (Reaktionsschema am Beispiel von Methyltrimethoxysilan):

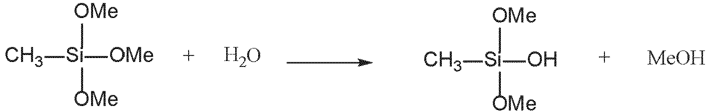

In Abhängigkeit von der eingesetzten Menge an Wasser kann die Hydrolyse-Reaktion auch mehrfach pro eingesetztem C₁-C₆-Alkoxy-Silan stattfinden: 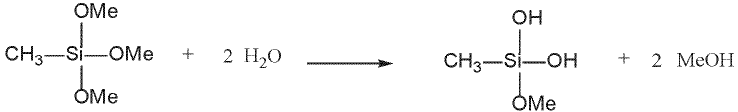 bzw.

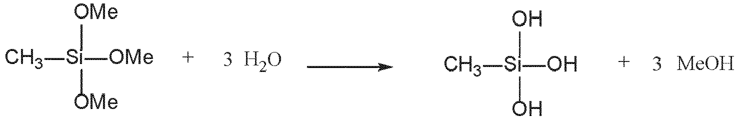

Mögliche Kondensationsreaktionen sind beispielsweise (gezeigt anhand des Gemisches (3-Aminopropyl)triethoxysilan und Methyltrimethoxysilan): 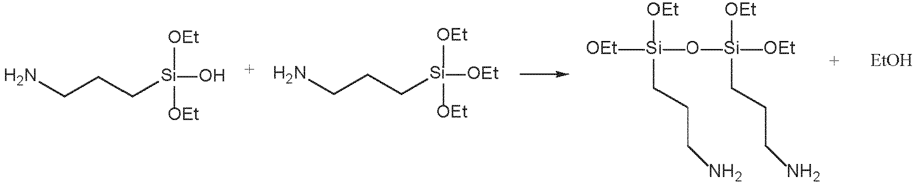 und/oder 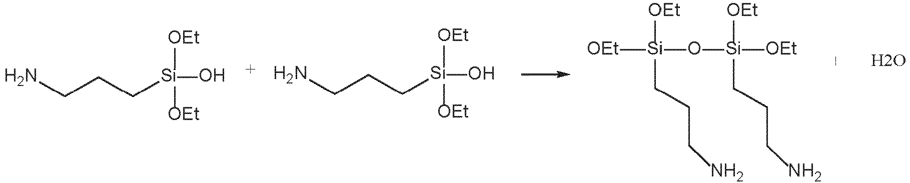 und/oder 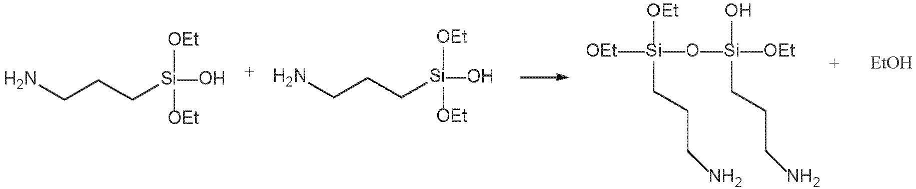 und/oder 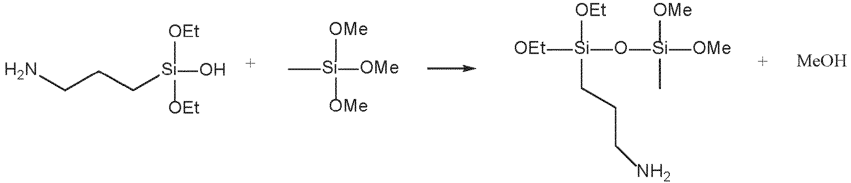 und/oder 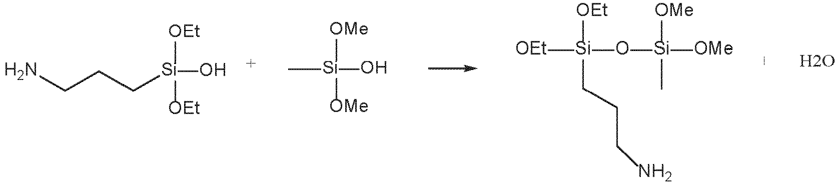 und/oder 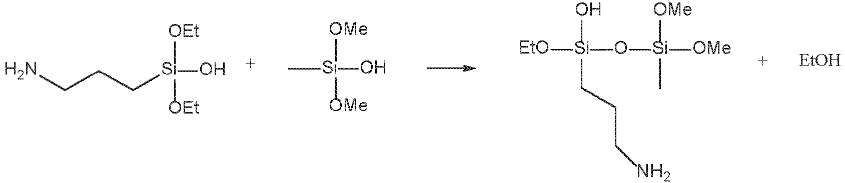 und/oder

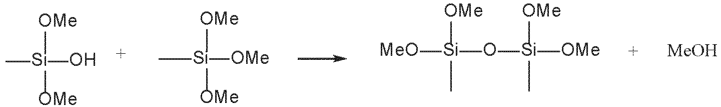

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt.

Die erfindungsgemäße Zusammensetzung (A) kann die organischen C₁-C₆-Alkoxysilane in verschiedenen Mengenanteilen enthalten. Diese bestimmt der Fachmann in Abhängigkeit von der gewünschten Dicke des Silan-Coatings auf dem Keratinmaterial und von der Menge des zu behandelnden Keratinmaterials.

Besonders lagerstabile Zubereitungen mit sehr gutem Färberesultat bei der Anwendung konnten erhalten werden, wenn die Zusammensetzung (A) - bezogen auf ihr Gesamtgewicht -die organischen C₁-C₆-Alkoxysilane und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

In einer weiteren Ausführungsform ist ein ganz besonders bevorzugtes Verfahren dadurch gekennzeichnet, dass die erste Zusammensetzung (A) - bezogen auf das Gesamtgewicht der Zusammensetzung (A) - e die organischen C₁-C₆-Alkoxysilane und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

### Weitere kosmetische Inhaltsstoffe in der Zusammensetzung (A)

Zusätzlich kann die Zusammensetzung (A) auch noch einen oder mehrere weitere kosmetische Inhaltsstoffe enthalten.

Bei den fakultativ in der Zusammensetzung (A) einsetzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Wie zuvor beschrieben wird der Gehalt an Polymeren in der Zusammensetzung (A) auf maximal 0,1 Gew.-% beschränkt bzw. bevorzugt ganz ausgeschlossen.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Als ganz besonders bevorzugt hat es sich in diesem Zusammenhang erwiesen, in der Zusammensetzung (A) einen kosmetischen Inhaltsstoff aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan einzusetzen.

In einer weiteren ganz besonders bevorzugten Ausführungsform ist ein erfindungsgemäßes Verfahren dadurch gekennzeichnet, das die erste Zusammsensetzung (A) mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

Hexamethyldisiloxan besitzt die CAS-Nummer 107-46-0 und kann beispielsweise bei Sigma-Aldrich kommerziell erworben werden.

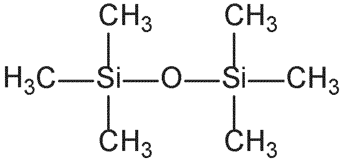

Octamethyltrisiloxan besitzt die CAS-Nummer 107-51-7 und ist ebenfalls bei Sigma-Aldrich kommerziell erhältlich.

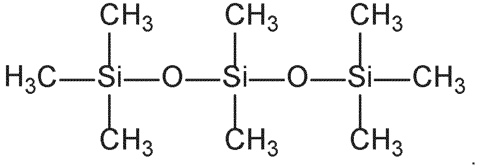

Decamethyltetrasiloxan trägt die CAS-Nummer 141-62-8 und ist ebenfalls bei Sigma-Aldrich kommerziell erhältlich.

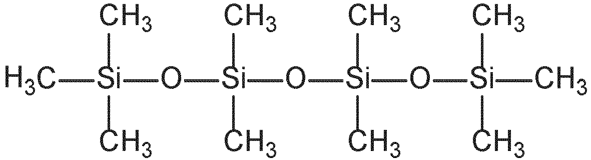

Hexamethylcyclotrisiloxan besitzt die CAS-Nr. 541-05-9.

Octamethylcyclotetrasiloxan besitzt die CAS-Nr. 556-67-2.

Decamethylcyclopentasiloxan besitzt die CAS-Nr. 541-02-6.

Als ganz besonders bevorzugt hat sich der Einsatz von Hexamethyldisiloxan in der Zusammensetzung (A) herausgestellt. Besonders bevorzugt ist Hexamethyldisiloxan - bezogen auf das Gesamtgewicht der Zusammensetzung (A) - in Mengen von 1,0 bis 20,0 Gew.-%, bevorzugt von 1,3 bis 10,0 Gew.-%, weiter bevorzugt von 1,6 bis 5,0 Gew.-% und ganz besonders bevorzugt von 2,0 bis 4,0 Gew.-% in der Zusammensetzung (A) enthalten.

### Wassergehalt (A1) in der Zusammensetzung (A)

Das erfindungsgemäße Verfahren ist gekennzeichnet durch die Anwendung einer ersten Zusammensetzung (A) auf dem keratinischen Material.

Unter der Zusammensetzung (A) wird im Rahmen der vorliegenden Erfindung eine anwendungsbereite Zusammensetzung, die in ihrer vorliegenden Ausgestaltungsform auf die Keratinmaterialein, insbesondere auf die Haare, appliziert werden kann.

Im Rahmen des erfindungsgemäßen Verfahrens kann die Zusammensetzung (A) entweder in ihrer vorliegenden Form in einem Container bereitgstellt werden. Mit den C₁-C₆-Alkoxy-Silanen enthält die Zusammensetzung (A) jedoch sehr reaktive Verbindungen. Zur Vermeidung von Problemen im Zusammenhang mit der Lagerstabilität ist es jedoch besonders bevorzugt, die anwendungsbereite und reaktive Zusammensetzung (A) erst kurz vor der Anwendung durch Vermischen von zwei oder mehrern lagerstabilien Zusammensetzungen herzustellen. Beispielsweise kann die anwendungsbereite Zusammensetzung (A) durch Vermischen von einem wassermen Silan-Blend (A-I), welcher die organischen C₁-C₆-Alkoxy-Silane in aufkonzentrierter Form enthält, und einer wasserreichen Träger-Formulierung (A-II), welche beispielsweise ein Gel, eine Lotion oder ein tensidisches System darstellen kann, hergestellt werden.

Die anwendungsbereite Zusammensetzung (A) besitzt dementsprechend bevorzugt einen höheren Wassergehalt, der - bezogen auf das Gesamtgewicht der Zusammensetzung (A) - im Bereich von 50,0 bis 90,0 Gew.-%, bevorzugt von 55,0 bis 90,0 Gew.-%, weiter bevorzugt 60,0 bis 90,0 Gew.-% und besonders bevorzugt 70,0 bis 90,0 Gew.-% liegen kann.

Im Rahmen einer weiteren Ausführungsform ist ein erfindungsgemäßes Verfahren dadurch gekennzeichnet, dass die erste Zusammensetzung (A) - bezogen auf das Gesamtgewicht der Zusammensetzung (A) - 50,0 bis 90,0 Gew.-%, bevorzugt von 55,0 bis 90,0 Gew.-%, weiter bevorzugt 60,0 bis 90,0 Gew.-% und besonders bevorzugt 70,0 bis 90,0 Gew.-% Wasser enthält.

### pH-Wert der Zusammensetzungen (A)

In weiteren Versuchen hat sich herausgestellt, dass die pH-Werte der Zusammensetzung (A) einen Einfluss auf die bei der Färbung erhaltenen Farbintensitäten nehmen können. Hierbei wurde gefunden, dass sich insbesondere alkalische pH-Werte vorteilhaft auf die im Verfahren erzielbare Färbeleistung auswirken.

Aus diesem Grund ist es bevorzugt, dass die Zusammensetzungen (A) einen pH-Wert von 7,0 bis 12,0, bevorzugt von 7,5 bis 11,5, weiter bevorzugt von 8,0 bis 11,0 und ganz besonders bevorzugt von 8,0 bis 10,5 besitzt.

Die Messung des pH-Wertes kann mit den üblichen aus dem Stand der Technik bekannten Methoden wie beispeislweise der pH-Wert Messung mittels Glaselektroden über Einstabmessketten oder aber über pH-Indikator-Papier erfolgen.

In einer weiteren ganz besonders bevorzugten Ausführungsform ist ein erfindungsgemäßes Verfahren, dadurch gekennzeichnet, dass die Zusammensetzung (A) einen pH-Wert von 7,0 bis 12,0, bevorzugt von 7,5 bis 11,5, weiter bevorzugt von 8,0 bis 11,0 und ganz besonders bevorzugt von 8,0 bis 10,5 besitzt.

Zur Einstellung der vorgenannten pH-Werte können die Alkalisierungsmittel verwendet werden, die auch zur Einstellung des pH-Wertes der Zusammensetzung (B) Einsatz finden können.

### Aminosäuren, Proteinhydrolysate und/oder Proteine in der Zusammensetzung (B)

Das erfindungsgemäße Verfahren umfasst die Anwendung einer zweiten Zusammensetzung (B) auf dem Keratinmaterial. Hierbei ist die Zusammensetzung (B) dadurch gekennzeichnet, dass sie mindestens eine Verbindung enthält, die ausgewählt ist aus der Gruppe aus Aminosäuren und/oder Proteinhydrolysaten.

Unter einer Aminosäure ist eine chemische Verbindung mit einer Aminogruppe und einer Carbonsäuregruppe zu verstehen. Zur Klasse der Aminosäuren zählen organische Verbindungen, die zumindest eine Aminogruppe (-NH₂ bzw. substituiert -NR₂) und eine Carboxygruppe (-COOH) als funktionelle Gruppen enthalten, also Strukturmerkmale der Amine und der Carbonsäuren aufweisen. Chemisch lassen sie sich nach der Stellung ihrer Aminogruppe zur Carboxygruppe unterscheiden - steht die Aminogruppe am C_{α}-Atom unmittelbar benachbart zur endständigen Carboxygruppe, nennt man dies α-ständig und spricht von α-Aminosäuren. Bevorzugt werden Carbonsäuren mit einer Gesamtzahl an C-Atomen von C2-20, bevorzugter von C2-15, besonders bevorzugt von C2-10 eingesetzt.

Bevorzugte Aminosäuren sind ausgewählt aus Arginin, Lysin, Histidin, Asparagin, Glutamin, Cystein, Methionin, Tryptophan, Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren.

Chirale Aminosäuren besitzen ein sterogenes Zentrum und können in spiegelbildlichen Formen auftregen. Beipsielsweise tritt Arginin in Form des L-Arginins und des D-Argnins auf. Sowohl die L-Form einer Aminosäure als auch ihre D-Form sowie die Gemische hiervon sind von der vorliegenden Erfindung umfasst. Im Rahmen der vorliegenden Erfindung können demnach beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Im Rahmen einer weiteren besonders bevorzugten Ausführungsform ist ein erfindungsgemäßes Verfahren dadurch gekennzeichnet, dass die zweite Zusammensetzung (B) mindestens eine Aminosäure enthält, die ausgewählt ist aus der Gruppe aus Arginin, Lysin, Histidin, Asparagin, Glutamin, Cystein, Methionin, Tryptophan, Serin, Alanin, Asparaginsäure, Glutaminsäure, Glycin, Isoleucin, Leucin, Phenylalanin, Prolin, Threonin, Tyrosin und Valin.

Die besten Ergebnisse wurden mit Arginin erhalten.

Im Rahmen einer weiteren besonders bevorzugten Ausführungsform ist ein erfindungsgemäßes Verfahren dadurch gekennzeichnet, dass die zweite Zusammensetzung (B) Arginin enthält.

Für die Erzielung möglichst guter Waschechtheiten werden die Aminosäure(n) in der Zusammensetzung (B) bevorzugt in bestimmten Mengenbereichen eingesetzt. Es hat sich als besonders vorteilhaft herausgestellt, wenn die Zusammensetzung (B) - bezogen auf das Gesamtgewicht der Zusammensetzung (B) eine oder mehrere Aminosäuren in einer Gesamtmenge von 0,1 bis 20,0 Gew.-%, bevorzugt 0,5 bis 10,0 Gew.-% enthält.

Im Rahmen einer weiteren besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - eine oder mehrere Aminosäuren in einer Gesamtmenge von 0,1 bis 20,0 Gew.-%, bevorzugt 0,5 bis 10,0 Gew.-% enthält.

Weiterhin gute Färbeergebnisse mit hoher Farbintensität und zusätzlich verbesserter Waschechtheit konnten erzielt werden, wenn in der Zusammensetzung (B) zusätzlich zu oder anstatt der Aminosäure mindestens ein Proteinhydrolysat eingesetzt wurde.

Proteinhydrolysate sind erfindungsgemäß Abbauprodukte von Proteinen, welche durch saure, basische oder enzymatische Reaktion hergestellt werden. Aufgrund des Herstellungsprozesses weisen Proteinhydrolysate eine Verteilung des Molekulargewichtes auf. Zu den erfindungsgemäßen Proteinhydrolysaten sind auch Oligopeptide zu zählen, da diese ebenfalls durch entsprechende Reaktionen aus Proteinen hergestellt werden können. Einzelne Aminosäuren, welche als diskrete Einzelverbindung vorliegen, zählen erfindungsgemäß nicht zu den Proteinhydrolysaten im Sinne dieser Erfindung. Erfindungsgemäß können Proteinhydrolysate sowohl pflanzlichen als auch tierischen oder marinen oder synthetischen Ursprungs eingesetzt werden.

Unter Proteinhydrolysaten im Sinne der vorliegenden Erfindung werden höchstens oligomere Verbindungen verstanden, die sich aus maximal 10 Aminosäuren zusammensetzen.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), ProSina^{®} (Croda) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Moringa-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda), Crotein^{®} (Croda) und Puricare^{®} LS 9658 von der Fa. Laboratoires Sérobiologiques erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Weiterhin sind kationisierte Proteinhydrolysate zu den Proteinhydrolysaten zu zählen, wobei das zugrundeliegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt.

Gute Ergebnisse konnten beobachtet werden, wenn die Zusammensetzung (B) mindestens ein Proteinhydrolysat enthielt, das ausgewählt ist aus den Hydrolysaten des Elastins, des Kollagens, des Keratins, der Seide, des Milcheiweißes, sowie der Soja-, Mandel-, Erbsen-, Moringa-, Kartoffel- und Weizenproteinhydrolysate.

Im Rahmen einer weiteren besonders bevorzugten Ausführungsform ist ein erfindungsgemäßes Verfahren dadurch gekennzeichnet, dass die zweite Zusammensetzung (B) ein Proteinhydrolysat enthält, das ausgewählt ist aus der Gruppe aus Proteinhydrolysaten des Elastins, des Kollagens, des Keratins, der Seide, des Milcheiweißes, Soja-, Mandel-, Erbsen-, Moringa-, Kartoffel- und Weizenproteinhydrolysaten.

Für die Erzielung möglichst guter Waschechtheiten werden das bzw. die Proteinhydrolysat in der Zusammensetzung (B) bevorzugt in bestimmten Mengenbereichen eingesetzt. Es hat sich als besonders vorteilhaft herausgestellt, wenn die Zusammensetzung (B) - bezogen auf das Gesamtgewicht der Zusammensetzung (B) eine oder mehrere Proteinhydrolysate in einer Gesamtmenge von 0,1 bis 20,0 Gew.-%, bevorzugt 0,5 bis 10,0 Gew.-% enthält.

Im Rahmen einer weiteren besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - eine oder mehrere Proteinhydrolysate in einer Gesamtmenge von 0,1 bis 20,0 Gew.-%, bevorzugt 0,5 bis 10,0 Gew.-% enthält.

Ebenfalls erfindungsgemäße Proteinhydrolysate sind Oligopeptide. Oligopeptide können in den erfindungsgemäßen Haarbehandlungsmitteln aufgrund ihrer definierten Aminosäuresequenz bevorzugt sein.

Ein Oligopeptid, welches mindestens eine Aminosäuresequenz Glu-Glu-Glu 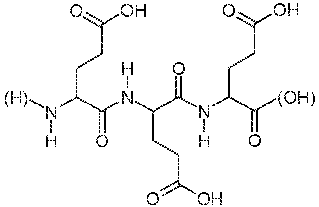 aufweist, wobei die Amino-Gruppe frei oder protoniert und die Carboxy-Gruppen frei oder deprotoniert vorliegen können, kann erfindungsgemäß besonders bevorzugt sein. In dieser wie in allen nachstehenden Formeln bedeutet das eingeklammerte Wasserstoffatom der Aminogruppe ebenso wie die eingeklammerte Hydroxygruppe der Säurefunktion, daß die betreffenden Gruppen als solche vorhanden sein können (dann handelt es sich um ein Oligopeptid mit der betreffenden Anzahl an Aminosäuren wie in der vorstehenden Formel, oder aber, daß die Aminosäuresequenz in einem Oligopeptid vorliegt, das noch weitere Anminosäuren umfaßt - je nachdem, wo die weitere(n) Aminosäure(n) gebunden ist/sind, sind die eingeklammerten Bestandteile der o.g. Formel durch den/die weiteren Aminosäurerest(e) ersetzt.

Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen. In erfindungsgemäß bevorzugten Haarbehandlungsmitteln umfaßt das Oligopeptid 5 bis 15 Aminosäuren, vorzugsweise 6 bis 13 Aminosäuren, besonders bevorzugt 7 bis 12 Aminosäuren und insbesondere 8, 9 oder 10 Aminosäuren. Je nachdem, ob weitere Aminosäuren an die Sequenz Glu-Glu-Glu gebunden sind und je nach Art dieser Aminosäuren kann die Molmasse des in den erfindungsgemäßen Mitteln enthaltenen Oligopeptids variieren. Erfindungsgemäß bevorzugte Haarbehandlungsmittel sind dadurch gekennzeichnet, daß das Oligopeptid eine Molmasse von 650 bis 3000 Da, vorzugsweise von 750 bis 2500 Da, besonders bevorzugt von 850 bis 2000 Da und insbesondere von 1000 bis 1600 Da aufweist. Wie aus der bevorzugten Anzahl von Aminisäuren in den Oligopeptiden und dem bevorzugten Molmassenbereich zu ersehen ist, werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind. Ein besonders bevorzugtes Oligopeptid enthält zusätzlich Tyrosin, das vorzugsweise über seine Säurefunktion an die Glu-Glu-Glu-Sequenz gebunden ist. Erfindungsgemäß bevorzugte Haarbehandlungsmittel sind daher dadurch gekennzeichnet, daß das in ihnen enthaltene Oligopeptid mindestens eine Aminosäuresequenz Tyr-Glu-Glu-Glu 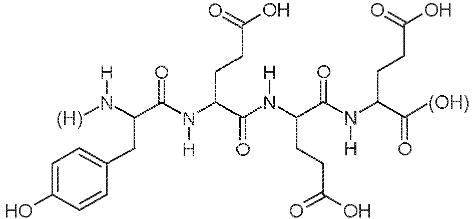 aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Erfindungsgemäß bevorzugte Haarbehandlungsmittel sind daher dadurch gekennzeichnet, daß das in ihnen enthaltene das Oligopeptid mindestens eine Aminosäuresequenz Glu-Glu-Glu-Ile 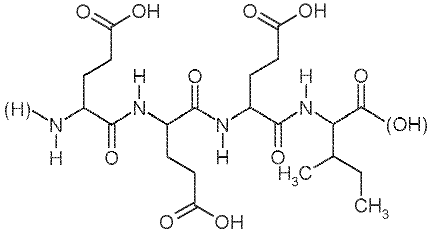 aufweist, wobei die Amino-Gruppe frei oder protoniert und die Carboxy-Gruppen frei oder deprotoniert vorliegen können.

Oligopeptide, die beide vorgenannten Aminosäuren (Tyrosin und Isoleucin) aufweisen, sind erfindungsgemäß bevorzugt. Besonders bevorzugt sind dabei erfindungsgemäße Haarbehandlungsmittel, bei denen das in ihnen enthaltene Oligopeptid mindestens eine Aminosäuresequenz Tyr-Glu-Glu-Glu-Ile 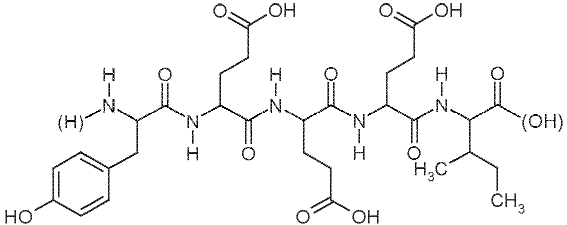 aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt

Noch weiter bevorzugte Oligopeptide enthalten zusätzlich Valin, das vorzugsweise an das Arginin gebunden vorliegt. Erfindungsgemäß weiter bevorzugte Haarbehandlungsmittel sind daher dadurch gekennzeichnet, daß das in ihnen enthaltene Oligopeptid mindestens eine Aminosäuresequenz Tyr-Glu-Glu-Glu-Ile-Arg-Val 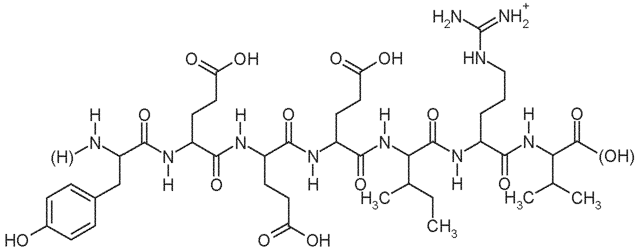 aufweist, wobei die Amino-Gruppen frei oder protoniert und die Carboxy-Gruppen frei oder deprotoniert vorliegen können. Noch weiter bevorzugte Oligopeptide enthalten zusätzlich Leucin, das vorzugsweise an das Valin gebunden vorliegt. Erfindungsgemäß weiter bevorzugte Haarbehandlungsmittel sind dadurch gekennzeichnet, daß das in ihnen enthaltene Oligopeptid mindestens eine Aminosäuresequenz Tyr-Glu-Glu-Glu-Ile-Arg-Val-Leu 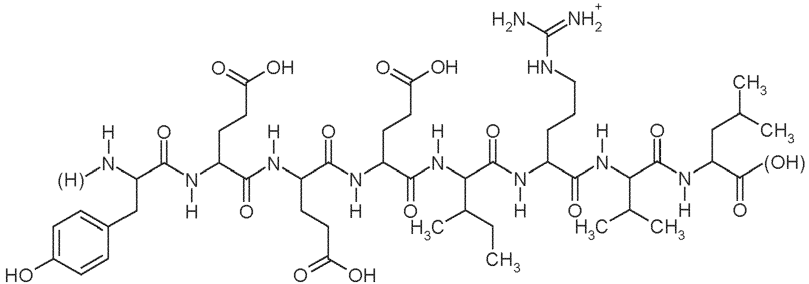 aufweist, wobei die Amino-Gruppen frei oder protoniert und die Carboxy-Gruppen frei oder deprotoniert vorliegen können.

Insbesondere bevorzugte Oligopeptide enthalten zusätzlich Leucin, das vorzugsweise an das Tyrosin gebunden vorliegt. Erfindungsgemäß weiter bevorzugte Haarbehandlungsmittel sind dadurch gekennzeichnet, daß das in ihnen enthaltene Oligopeptid mindestens eine Aminosäuresequenz Leu-Tyr-Glu-Glu-Glu-Ile-Arg-Val-Leu 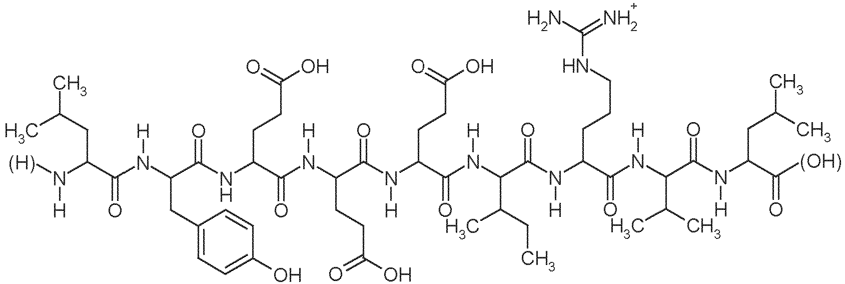 aufweist, wobei die Amino-Gruppen frei oder protoniert und die Carboxy-Gruppen frei oder deprotoniert vorliegen können.

### Wassergehalt der Zusammensetzung (B)

Die Zusammensetzung (B) enthält das oder die Aminosäuren, Proteinhydrolysate und/oder Proteine in einem kosmetischen Träger, bevorzugt in einem wässrigen kosmetischen Träger.

In diesem Zusammenhang hat es sich als bevorzugt herausgestellt, wenn die Zusammensetzung (B) - bezogen auf das Gesamtgewicht der Zusammensetzung (B) - 5,0 bis 99,0 Gew.-%, bevorzugt 15,0 bis 97,0 Gew.-%, weiter bevorzugt 25,0 bis 97,0 Gew.-% , nocht weiter bevorzugt 35,0 bis 97,0 Gew.-% und ganz besonders bevorzugt 45,0 bis 97,0 Gew.-% Wasser enthält.

Im Rahmen einer weiteren Ausführungsform ist ein erfindungsgemäßes Verfahren dadurch gekennzeichnet dass die zweite Zuammensetzung (B) - bezogen auf das Gesamtgewicht der Zusammensetzung (B) - 5,0 bis 99,0 Gew.-%, bevorzugt 15,0 bis 97,0 Gew.-%, weiter bevorzugt 25,0 bis 97,0 Gew.-% , nocht weiter bevorzugt 35,0 bis 97,0 Gew.-% und ganz besonders bevorzugt 45,0 bis 97,0 Gew.-% Wasser enthält.

### Weitere kosmetische Inhaltsstoffe in der Zusammensetzung (B)

Zusätzlich kann die Zusammensetzung (B) auch noch einen oder mehrere weitere kosmetische Inhaltsstoffe enthalten.

Bei den fakultativ in der Zusammensetzung (B) einsetzbaren kosmetischen Inhaltsstoffen kann es sich um alle geeigneten Bestandteile handeln, um dem Mittel weitere positive Eigenschaften zu verleihen. Beispielsweise können in der Zusammensetzung (B) ein Lösungsmittel, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel und der Pflanzenextrakte enthalten sein.

Wie zuvor beschrieben wird der Gehalt an Polymeren in der Zusammensetzung (B) auf maximal 0,1 Gew.-% beschränkt bzw. bevorzugt ganz ausgeschlossen.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

### pH-Wert der Zusammensetzungen (B)

In weiteren Versuchen hat sich herausgestellt, dass auch die pH-Werte der Zusammensetzung (B) einen Einfluss auf die bei der Färbung erhaltenen Farbintensitäten und Waschechtheiten nehmen können. Hierbei wurde gefunden, dass sich insbesondere alkalische pH-Werte vorteilhaft auf die im Verfahren erzielbare Färbeleistung auswirken.

Aus diesem Grund besitzt die Zusammensetzungen (B) einen pH-Wert von 8,0 bis 10,5.

Die Messung des pH-Wertes kann mit den üblichen aus dem Stand der Technik bekannten Methoden wie beispeislweise der pH-Wert Messung mittels Glaselektroden über Einstabmessketten oder aber über pH-Indikator-Papier erfolgen.

Zur Einstellung dieses alkalischen pH-Wertes kann es erforderlich werden, der Reaktionsmischung ein Alkalisierungsmittel und/oder Acidifizierungsmittel zuzusetzen. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Aminosäuren eingesetzt werden.

Alkanolamine können ausgewählt werden aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Werden in der Zusammensetzung (B) basische Aminsäuren eingesetzt, so ist es ebenfalls möglich, die Einstellung des pH-Wertes durch Zugabe der basischen Aminosäuren selbst vorzunehmen. Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch anorganische Alkalisierungsmittel eingesetzt werden. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierungsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

### Verzicht auf Polymere in der Zusammensetzung (A) und (B)

Das erfindungsgemäße Verfahren ist dadurch gekennzeichnet, dass die im Verfahren eingesetzten Zusammensetzungen (A) und (B) im wesentlichen frei von Polymeren sind.

Kennzeichnend für die Zusammensetzung (A) ist, dass der Gesamtgehalt aller in der Zusammensetzung (A) enthaltenen Polymere - bezogen auf das Gesamtgewicht der Zusammensetzung (A) - unterhalb von 0,1 Gew.-% liegt.

Auch die Zusammensetzung (B) ist dadurch gekennezeichnet, dass der Gesamtgehalt aller in der Zusammensetzung (B) enthaltenen Polymere - bezogen auf das Gesamtgewicht der Zusammensetzung (B) - unterhalb von 0,1 Gew.-% liegt.

Durch Verzicht auf Polymere in den beiden Zusammensetzungen (A) und (B) wurde eine Möglichkeit gefunden, keratinisches Material, insbesondere keratinische Fasern wie Haare, wiederholten Färbeprozessen zu unterziehen, ohne dass der Anwender bei der Folgeanwendung eine Verminderung der Farbintensität wahrnimmt.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers (c) nicht mehr als 10⁷ g/mol, bevorzugt nicht mehr als 10⁶ g/mol und besonders bevorzugt nicht mehr als 10⁵ g/mol beträgt.

Die im erfindungsgemäßen Verfahren angewendete Zusammensetzung (A) kann das Kondensationsprodukt eines organischen C₁-C₆-Alkoxy-Silans enthalten. Bei den Kondensationsprodukten (A1) handelt es sich um oligomere Verbindungen, die nicht unter die Definition eines Polymers fallen. Allerdings kann nicht vollständig ausgeschlossen werden, dass unter außergewöhnlichen Lagerbedigungen, wie beispielsweise bei besonders langen Lagerzeiten oder sehr hohen Temperaturen, in der Zusammensetzung (A) ein sehr geringer Anteil der C₁-C₆-Alkoxy-Silane nicht nur zu oligomeren, sondern zu polymeren Verbindungen durch kondensiert.

Weiterhin kann die im erfindungsgemäßen Verfahren angewendete Zusammensetzung (B) ein Proteinhydrolysat enthalten. Auch bei den Proteinhydrolysaten (B1), handelt es sich um oligomere Verbindungen, die nicht unter die Definition eines Polymers fallen. Im Fall der Proteinhydrolysate (B1) kann jedoch nicht kategorisch ausgeschlossen werden, dass mit Bezug eines entsprechenden käuflichen Proteinhydrolysats (B1) ein Rohstoff geliefert wird, der unter Umständen als Nebenprodukt in sehr geringen Mengen auch Anteile eines hochmolekularen Proteins enthält.

Wenn der Gesamtgehalt aller in der Zusammensetzung (A) enthaltenen Polymere - bezogen auf das Gesamtgewicht der Zusammensetzung (A) - auf einen Wert unterhalb von 0,01 Gew.-% beschränkt wurde, konnten die Ergebnisse noch weiter verbessert werden.

In einer weiteren ganz besonders bevorzugten Ausführungsform ist ein erfindungsgemäßes Verfahren daher dadurch gekennzeichnet, dass der Gesamtgehalt aller in der Zusammensetzung (A) enthaltenen Polymere - bezogen auf das Gesamtgewicht der Zusammensetzung (A) - unterhalb von 0,01 Gew.-% liegt.

Gleiches gilt auch für die Zusammensetzung (B). Wenn der Gesamtgehalt aller in der Zusammensetzung (B) enthaltenen Polymere - bezogen auf das Gesamtgewicht der Zusammensetzung (B) - auf einen Wert unterhalb von 0,01 Gew.-% beschränkt wurde, konnten die Ergebnisse noch weiter verbessert werden.

In einer weiteren ganz besonders bevorzugten Ausführungsform ist ein erfindungsgemäßes Verfahren daher dadurch gekennzeichnet, dass der Gesamtgehalt aller in der Zusammensetzung (B) enthaltenen Polymere - bezogen auf das Gesamtgewicht der Zusammensetzung (B) - unterhalb von 0,01 Gew.-% liegt.

Besonders bevorzugt sind die Zusammensetzungen (A) und (B) im wesentlichen frei von filmbildenden Verbindungen. Die filmbildenden Polymere können hydrophil oder hydrophob sein.

Im Rahmen einer weiteren Ausführungsform kann es bevorzugt sein, in der Zubereitung (A) und/oder (B), auf den Einsatz eines hydrophobes, filmbildenden Polymers zu verzichten.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Gilmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Als weitere filmbildende hydrophobe Polymere können die Verbindungen genannt werden, die aus der Gruppe der synthetischen Polymere, der durch radikalische Polymerisation erhältlichen Polymere oder der natürlichen Polymere ausgewählt werden.

Weitere filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende hydrophobe Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Als anionische Polymere können zum Beispiel die Copolymere aus Acrylsäure, Methacrylsäure oder deren C₁-C₆-Alkylestern, genannt werden, wie sie unter der INCI-Deklaration Acrylates Copolymere vertrieben werden. Ein Handelsprodukt ist beispielsweise Aculyn^{®} 33 der Firma Rohm & Haas. Weitere Polymere sind Copolymere aus Acrylsäure, Methacrylsäure oder deren C₁-C₆-Alkylestern und den Estern einer ethylenisch ungesättigten Säure und einem alkoxylierten Fettalkohol.

Auf dem Markt befindliche Polymere sind beispielsweise Aculyn^{®} 22 (Acrylates/Steareth-20 Methacrylate Copolymer), Aculyn^{®} 28 (Acrylates/Beheneth-25 Methacrylate Copolymer), Structure 2001^{®}(Acryla-tes/Steareth-20 Itaconate Copolymer), Structure 3001^{®}(Acrylates/Ceteth-20 Itaconate Copolymer), Structure Pluso (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin können genannte werden die Copolymere Octylacrylamid/acrylates/ butylaminoethylmethacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als Polymere auf der Basis von Olefinen können beispielsweise genannt werden die Homo- und Copolymere des Ethylens, des Propylens, des Butens, des Isoprens und des Butadiens.

Weitere filmbildende hydrophobe Polymere sind die Block-Copolymere, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Alle diese Polymere werden im Rahmen des erfindungsgemäßen Verfahrens in den Zusammensetzungen (A) und (B) höchstens in den zuvor beschriebenen Maximalmengen eingesetzt.

Weitere Polymere sind hydrophile, filmbildende Polymere.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint markoskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere ganennt werden.

Zum Beipsiel können filmbildende, hydrophile Polymere aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummen, der Polysaccharide und/oder der Acrylamid-(Co)Polymere ausgewählt werden.

Als filmbildendes, hydrophiles Polymer kann auch Polyvinylpyrrolidon (PVP) genannt werden.

Entsprechende Polyvinylpyrrolidone sind beispielsweise unter der Bezeichnung Luviskol^{®} K von der BASF SE erhältlich, insbesondere Luviskol^{®} K 90 oder Luviskol^{®} K 85 der Firma BASF SE.

Als weiteres Polyvinylpyrrolidon (PVP) kann auch das Polymer PVP K30 genannt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Als weitere filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73 sind jeweils Vinylpyrrolidon/Vinylacetat-Copolymere.

In die Gruppe der Vinylpyrrolidon-haltigen Copolymere fallen auch Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

Ein weiteres Copolymer des Vinylpyrrolidons ist das unter der INCI Bezeichnung Maltodextrin/VP Copolymer bekannte Polymer.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Entsprechende nichtionische, filmbildende, hydrophile Polymere sind ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C₁ bis C₄)-Alkylamino-(C₂ bis C₄)-alkylacrylamid.

Kommen Copolymere aus N-Vinylpyrrolidon und Vinylacetat zum Einsatz, kann das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegen. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches ePolymer ist das Copolymer aus N-Vinylpyrrolidon, N-Vinylcaprolactam, N-(3-Dimethylaminopropyl)methacrylamid und 3-(Methacryloylamino)propyl-lauryl-dimethyl-ammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch, Molekulargewicht 350000) von der Firma ISP vertrieben wird.

Weitere filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich.

Als anionische filmbildende, hydrophile Polymere können zum Beispiel Acrylsäure-Polymere genannt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum .

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Filmbildende, hydrophile Polymere aus der Gruppe der Acrylamde sind beispielsweise Polymere, welche ausgehend von Monomeren der (Methy)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Vernetzte und ganz oder teilweise neutraliserte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres Polymer dieses Typs ist das der Firma Clamant unter dem Handelsnamen Hostacerin AMPS vertriebene vernetzte Poly-2-acrylamido-2methyl-propanesulphonsäure-Polymer, das teilweise mit Ammoniak neutralisiert ist.

Alle diese Polymere werden im Rahmen des erfindungsgemäßen Verfahrens in den Zusammensetzungen (A) und (B) höchstens in den zuvor beschriebenen Maximalmengen eingesetzt.

### Verfahren zum Färben von Keratinmaterial

Im Zuge der zu dieser Erfindung führenden Arbeiten wurde beobachtet, dass die Anwendung der Zusammensetzungen (A) und (B) in einem Färbeverfahren zur Färbungen mit besonders hoher Farbintensität und guter Waschechtheit führt. Insbesondere bei wiederholter Anwendungkonnten gute Ergebnisse erhalten werden.

Handelt es sich bei dem erfindungsgemäßen Verfahren um ein Verfahren zum Färben von Keratinmaterial, so umfasst mindestens ein Vefahrensschritt die Anwendung mindestens einer farbgebenden Verbindung, insbesondere mindestens eines Pigments. Hierbei ist es möglich, das Pigment mit in die Zusammensetzung (A) einzuarbeiten. Ebenfalls möglich ist es, der Zusammensetzung (B) mindestens ein Pigment hinzuzusetzen. Darüberhinaus ist es weiterhin erfindungsgemäß, wenn die farbgebende Verbindung, insbesondere das Pigment, in eine dritte Zusammensetzung (C) eingearbeitet wird, die biespielsweise vor oder nach der Zusammensetzung (A) auf das Keratinmaterial appliziert werden kann.

Es hat sich als ganz besonders bevorzugt herausgestellt, wenn die erste Zusammensetzung (A) zusätzlich mindestens eine farbgebende Verbindung aus der Gruppe der Pigmente und der direktziehenden Farbstoffe enthält.

Im Rahmen einer explizit ganz besonders bevorzugten Ausführungsform ist ein erfindungsgemäßes Verfahren dadurch gekennzeichnet, dass die erste Zusammensetzung (A) mindestens eine farbgebende Verbindung as der Gruppe der Pigmente und/oder der direktziehenden Farbstoffe enthält.

Weiterhin hat es sich auch als ganz besonders bevorzugt herausgestellt, wenn die zweite Zusammensetzung (B) zusätzlich mindestens eine farbgebende Verbindung aus der Gruppe der Pigmente und der direktziehenden Farbstoffe enthält.

Im Rahmen einer explizit ganz besonders bevorzugten Ausführungsform ist ein erfindungsgemäßes Verfahren dadurch gekennzeichnet, dass die zweite Zusammensetzung (B) mindestens eine farbgebende Verbindung as der Gruppe der Pigmente und/oder der direktziehenden Farbstoffe enthält.

Das oder die farbgebenden Verbindungen können vorzugsweise ausgewählt werden aus der Pigmente, der direktziehenden Farbstoffe, wobei es sich bei den direktziehenden Farbstoffen auch um photochromen Farbstoffe und thermochromen Farbstoffe handeln kann.

Ganz besonders bevorzugt enthält die Zusammensetzung (A) und/oder die Zusammensetzung (B) mindestens ein Pigment.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte farbgebende Verbindungen aus der Gruppe der Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Im Rahmen einer ganz besonders bevorzugten Ausführungsform ist ein erfindungsgemäßes Verfahren dadurch gekennzeichnet, dass die Zusammensetzung (A) und/oder die Zusammensetzung (B) mindestens eine farbgebende Verbindung aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist die erfindungsgemäße Zusammensetzung (A) und/oder die Zusammensetzung (B) dadurch gekennzeichnet, dass sie mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbgebenden Verbindungen auf Mica- oder Glimmerbasis, die mit mindestens einem Metalloxid und/oder einem Metalloxychlorid beschichtet sind.

In einer weiteren bevorzugten Ausführungsform ist eine erfindungsgemäße Zusammensetzung (A) und/oder Zusammensetzung (B) dadurch gekennzeichnet, dass sie mindestens eine farbgebende Verbindung enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann Zusammensetzung (A) und/oder die Zusammensetzung (B) auch ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass die Zusammensetzung (A) und/oder die Zusammensetzung (B) mindestens eine farbgebende Verbindung aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Zur Färbung des Keratinmaterials können auch Pigmente mit einer bestimmten Formgebung eingesetzt worden sein. Beispielsweise kann ein Pigment auf Basis eines lamellaren und/oder eines lentikularen Substratplättchens eingesetzt werden. Weiterhin ist auch die Färbung auf Basis eines Substratplättchens möglich, welches ein Vakuum metallisiertes Pigment umfasst.

Die Substratplättchen dieses Typs weisen eine durchschnittliche Dicke von höchstens 50 nm, vorzugsweise weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Substratplättchen sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf.

Durch die geringe Dicke der Substratplättchen weist das Pigment ein besonders hohes Deckvermögen auf.

Die Substratplättchen sind monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können. Die Substratplättchen sind vorzugsweise homogen aufgebaut, d.h. dass innerhalb der Plättchen kein Konzentrationsgradient auftritt. Insbesondere sind die Substratplättchen nicht schichtartig aufgebaut und weisen keine darin verteilten Teilchen oder Partikel auf.

Die Größe des Substratplättchens kann auf den jeweiligen Anwendungszweck, insbesondere dem gewünschten Effekt auf dem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750, beträgt. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen können aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus Metall(legierung)en aufgebaut.

Als Metall kommt jedes für metallische Glanzpigmente geeignete Metall in Betracht. Derartige Metalle sind unter anderem Eisen und Stahl, sowie alle luft- und wasserbeständigen (Halb)metalle wie beispielsweise Platin, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Lamellare Substratplättchen zeichnen sich durch einen unregelmäßig strukturierten Rand aus und werden aufgrund ihres Erscheinungsbildes auch als "cornflakes" bezeichnet.

Aufgrund ihrer unregelmäßigen Struktur erzeugen Pigmente auf der Basis von lamellaren Substratplättchen einen hohen Anteil an Streulicht. Außerdem decken die Pigmente auf der Basis von lamellaren Substratplättchen die vorhandene Farbe eines keratinischen Materials nicht vollständig ab und es können beispielsweise Effekte analog zu einer natürlichen Ergrauung erzielt werden.

Lentikulare (= linsenförmige) Substratplättchen weisen einen im Wesentlichen regelmäßigen runden Rand auf und werden aufgrund ihres Erscheinungsbildes auch als "silverdollars" bezeichnet. Aufgrund ihrer regelmäßigen Struktur überwiegt bei Pigmenten auf Basis von lentikularen Substratplättchen der Anteil des reflektierten Lichts.

Vakuum metallisierte Pigmente (*vacuum metallized pigments,* VMP) können beispielsweise durch das Freisetzen von Metallen, Metalllegierungen oder Metalloxiden von entsprechend beschichteten Folien gewonnen werden. Sie zeichnen sich durch eine besonders geringe Dicke der Substratplättchen im Bereich von 5 bis 50 nm und durch eine besonders glatte Oberfläche mit erhöhter Reflektivität aus. Substratplättchen, welche ein im Vakuum metallisiertes Pigment umfassen, werden im Rahmen dieser Anmeldung auch als VMP-Substratplättchen bezeichnet. VMP-Substratplättchen aus Aluminium können beispielsweise durch Freisetzen von Aluminium von metallisierten Folien gewonnen werden.

Die Substratplättchen aus Metall oder Metalllegierung können passiviert sein, beispielsweise durch Eloxieren (Oxidschicht) oder Chromatieren.

Unbeschichtete lamellare, lentikulare und/oder VPM-Substratplättchen, insbesondere solche aus Metall oder Metalllegierung, reflektieren das einfallende Licht in hohem Maße und erzeugen einen Hell-Dunkel-Flop, aber keinen Farbeindruck.

Ein Farbeindruck kann beispielsweise aufgrund optischer Interferenzeffekte erzeugt werden. Derartige Pigmente können auf mindestens einfach beschichteten Substratplättchen beruhen. Diese zeigen Interferenzeffekte durch Überlagerung von verschieden gebrochenen und reflektierten Lichtstrahlen.

Entsprechend sind bevorzugte Pigmente, Pigmente auf Basis eines beschichteten lamellaren Substratplättchens. Das Substratplättchen weist vorzugsweise mindestens eine Beschichtung B aus einem hochbrechenden Metalloxid mit einer Beschichtungsdicke von mindestens 50 nm auf. Zwischen der Beschichtung B und der Oberfläche des Substratplättchens befindet sich vorzugsweise noch eine Beschichtung A. Gegebenenfalls befindet sich auf der Schicht B eine weitere Beschichtung C, die von der darunterliegenden Schicht B verschieden ist.

Als Materialien für die Beschichtungen A, B und C eignen sich alle Substanzen, die filmartig und dauerhaft auf die Substratplättchen aufgebracht werden können und, im Fall der Schichten A und B, die erforderlichen optischen Eigenschaften aufweisen. Allgemein ist eine Beschichtung eines Teils der Oberfläche der Substratplättchen ausreichend, um ein Pigment mit einem glänzenden Effekt zu erhalten. So können beispielsweise lediglich die obere und/oder untere Seite der Substratplättchen beschichtet sein, wobei die Seitenfläche(n) ausgespart sind. Vorzugsweise ist die gesamte Oberfläche der gegebenenfalls passivierten Substratplättchen, einschließlich der Seitenflächen, von Beschichtung B bedeckt. Die Substratplättchen sind also vollständig von Beschichtung B umhüllt. Dies verbessert die optischen Eigenschaften des Pigments und erhöht die mechanische und chemische Belastbarkeit der Pigmente. Das Vorstehende gilt auch für die Schicht A und vorzugsweise auch für die Schicht C, falls vorhanden.

Obwohl jeweils mehrere Beschichtungen A, B und/oder C vorhanden sein können, weisen die beschichteten Substratplättchen vorzugsweise jeweils nur eine Beschichtung A, B und, falls vorhanden, C auf.

Die Beschichtung B ist aus mindestens einem hochbrechenden Metalloxid aufgebaut. Hochbrechende Materialien weisen einen Brechungsindex von mindestens 1,9, bevorzugt mindestens 2,0 und besonders bevorzugt mindestens 2,4 auf. Vorzugsweise umfasst die Beschichtung B mindestens 95 Gew.-%, besonders bevorzugt mindestens 99 Gew.-% an hochbrechenden Metalloxid(en).

Die Beschichtung B weist eine Dicke von mindestens 50 nm auf. Vorzugsweise beträgt die Dicke von Beschichtung B nicht mehr als 400 nm, besonders bevorzugt höchstens 300 nm.

Für Beschichtung B geeignete hochbrechende Metalloxide sind vorzugsweise selektiv lichtabsorbierende (d.h. farbige) Metalloxide, wie beispielsweise Eisen(III)oxid (α- und γ-Fe2O3, rot), Cobalt(II)oxid (blau), Chrom(III)oxid (grün),Titan(III)oxid (blau, liegt üblicherweise im Gemisch mit Titanoxynitriden und Titannitriden vor) und Vanadium(V)oxid (orange) sowie deren Gemische. Es eignen sich auch farblose hochbrechende Oxide wie Titandioxid und/oder Zirkonoxid.

Beschichtung B kann einen selektiv absorbierenden Farbstoff enthalten, vorzugsweise 0,001 bis 5 Gew.-%, besonders bevorzugt 0,01 bis 1 Gew.-%, jeweils bezogen auf die Gesamtmenge der Beschichtung B. Geeignet sind organische und anorganische Farbstoffe, die sich stabil in eine Metalloxidbeschichtung einbauen lassen.

Die Beschichtung A weist vorzugsweise mindestens ein niedrigbrechendes Metalloxid und/oder Metalloxidhydrat auf. Vorzugsweise umfasst Beschichtung A mindestens 95 Gew.-%, besonders bevorzugt mindestens 99 Gew.-% niedrigbrechendes Metalloxid(hydrat). Niedrigbrechende Materialien weisen einen Brechungsindex von höchstens 1,8, bevorzugt höchstens 1,6 auf.

Zu den niedrigbrechenden Metalloxiden, die für die Beschichtung A geeignet sind, zählen beispielsweise Silicium(di)oxid, Siliciumoxidhydrat, Aluminiumoxid, Aluminiumoxidhydrat, Boroxid, Germaniumoxid, Manganoxid, Magnesiumoxid und deren Gemische, wobei Siliciumdioxid bevorzugt ist. Die Beschichtung A weist bevorzugt eine Dicke von 1 bis 100 nm, besonders bevorzugt 5 bis 50 nm, insbesondere bevorzugt 5 bis 20 nm, auf.

Vorzugsweise beträgt der Abstand zwischen der Oberfläche der Substratplättchen und der inneren Oberfläche von Beschichtung B höchstens 100 nm, besonders bevorzugt höchstens 50 nm, insbesondere bevorzugt höchstens 20 nm. Dadurch, dass die Dicke von Beschichtung A und somit der Abstand zwischen der Oberfläche der Substratplättchen und Beschichtung B im oben angegebenen Bereich liegt, kann sichergestellt werden, dass die Pigmente ein hohes Deckvermögen aufweisen.

Weist das Pigment auf Basis eines lamellaren Substratplättchens nur eine Schicht A auf, ist es bevorzugt, dass das Pigment ein lamellares Substratplättchen aus Aluminium und eine Schicht A aus Siliciumdioxid aufweist. Weist das Pigment auf Basis eines lamellaren Substratplättchens eine Schicht A und eine Schicht B auf, ist es bevorzugt, dass das Pigment ein lamellares Substratplättchen aus Aluminium, eine Schicht A aus Siliciumdioxid und eine Schicht B aus Eisenoxid aufweist.

Gemäß einer bevorzugten Ausführungsform weisen die Pigmente eine weitere Beschichtung C aus einem Metalloxid(hydrat), die von der darunterliegenden Beschichtung B verschieden ist, auf. Geeignete Metalloxide sind beispielsweise Silicium(di)oxid, Siliciumoxidhydrat, Aluminiumoxid, Aluminiumoxidhydrat, Zinkoxid, Zinnoxid, Titandioxid, Zirkonoxid, Eisen(III)oxid und Chrom(III)oxid. Bevorzugt ist Siliciumdioxid.

Die Beschichtung C weist vorzugsweise eine Dicke von 10 bis 500 nm, besonders bevorzugt 50 bis 300 nm auf. Durch das Vorsehen der Beschichtung C, beispielsweise auf Basis von TiO₂, kann eine bessere Interferenz erzielt werden, wobei ein hohes Deckvermögen gewährleistet bleibt.

Die Schichten A und C dienen insbesondere als Korrosionsschutz als auch der chemischen und physikalischen Stabilisierung. Besonders bevorzugt enthalten die Schichten A und C sind Siliciumdioxid oder Aluminiumoxid, die nach dem Sol-Gel-Verfahren aufgebracht werden. Dieses Verfahren umfasst das Dispergieren der unbeschichteten lamellaren Substratplättchen oder der bereits mit Schicht A und/oder Schicht B beschichteten lamellaren Substratplättchen in einer Lösung eines Metallalkoxids wie Tetraethylorthosilikat oder Aluminiumtriisopropanolat (üblicherweise in einer Lösung von organischem Lösungsmittel oder einer Mischung von organischem Lösungsmittel und Wasser mit mindestens 50 Gew.-% organisches Lösungsmittel wie ein C1 bis C4-Alkohol), und Zugabe einer schwachen Base oder Säure zur Hydrolysierung des Metallalkoxids, wodurch ein Film des Metalloxids auf der Oberfläche der (beschichteten) Substratplättchen entsteht.

Die Schicht B kann beispielsweise durch hydrolytische Zersetzung einer oder mehrerer organischer Metallverbindungen und/oder durch Fällung eines oder mehrerer gelöster Metallsalze sowie eine ggf. anschließende Nachbehandlung (zum Beispiel Überführen einer gebildeten hydroxidhaltigen Schichten in die Oxidschichten durch Tempern) hergestellt werden.

Obwohl jede der Beschichtungen A, B und/oder C aus einem Gemisch von zwei oder mehreren Metalloxid(hydrat)en aufgebaut sein kann, ist jede der Beschichtungen vorzugsweise aus einem Metalloxid(hydrat) aufgebaut.

Die Pigmente auf Basis von beschichteten lamellaren bzw. lentikularen Substratplättchen bzw. die Pigmente auf Basis von beschichteten VMP-Substratplättchen weisen vorzugsweise eine Dicke von 70 bis 500 nm, besonders bevorzugt 100 bis 400 nm, insbesondere bevorzugt 150 bis 320 nm, beispielsweise 180 bis 290 nm, auf. Aufgrund der geringen Dicke der Substratplättchen weist das Pigment ein besonders hohes Deckvermögen auf. Die geringe Dicke der beschichteten Substratplättchen wird insbesondere dadurch erzielt, dass die Dicke der unbeschichteten Substratplättchen gering ist, aber auch dadurch, dass die Dicken der Beschichtungen A und, falls vorhanden, C auf einen möglichst kleinen Wert eingestellt werden. Die Dicke von Beschichtung B bestimmt den Farbeindruck des Pigments.

Die Haftung und Abriebbeständigkeit von Pigmenten auf Basis von beschichteten Substratplättchen im keratinischen Material kann deutlich erhöht werden, in dem die äußerste Schicht, je nach Aufbau Schicht A, B oder C, zusätzlich durch organische Verbindung wie Silane, Phosphorsäureester, Titanate, Borate oder Carbonsäuren modifiziert wird. Dabei sind die organischen Verbindungen an die Oberfläche der äußersten, vorzugsweise Metalloxid-haltigen, Schicht A, B oder C gebunden. Die äußerste Schicht bezeichnet die Schicht, die räumlich am weitesten von dem lamellaren Substratplättchen entfernt ist. Bei den organischen Verbindungen handelt es sich vorzugsweise um funktionelle Silanverbindungen, die an die Metalloxid-haltige Schicht A, B oder C binden können. Hierbei kann es sich entweder um mono- als auch um bifunktionelle Verbindungen handeln. Beispiele für bifunktionelle organische Verbindungen sind Methacryloxypropenyltrimethoxysilan, 3-Methacryloxypropyltrimethoxysilan, 3- Acryloxypropyltrimethoxysilan, 2-Acryloxyethyltrimethoxysilan, 3-Methacryloxy- propyltriethoxysilan, 3-Acryloxypropyltrimethoxysilan, 2-Methacryloxyethyl- triethoxysilan, 2-Acryloxyethyltriethoxysilan, 3-Methacryloxypropyltris(methoxyethoxy)silan, 3-Methacryloxypropyltris(butoxyethoxy)silan, 3-Methacryloxy-propyltris(propoxy)silan, 3-Methacryloxypropyltris(butoxy)silan, 3-Acryloxy-propyltris(methoxyethoxy)silan, 3-Acryloxypropyltris(butoxyethoxy)silan, 3-Acryl-oxypropyltris(butoxy)silan, Vinyltrimethoxysilan, Vinyltriethoxysilan, Vinylethyl- dichlorsilan, Vinylmethyldiacetoxysilan, Vinylmethyldichlorsilan, Vinylmethyldiethoxysilan, Vinyltriacetoxysilan, Vinyltrichlorsilan, Phenylvinyldiethoxysilan, oder Phenylallyldichlorsilan. Des Weiteren kann eine Modifizierung mit einem monofunktionellen Silan, insbesondere eines Alkylsilans oder Arylsilans, erfolgen. Dieses weist nur eine funktionelle Gruppe auf, welche kovalent an die Oberfläche Pigments auf Basis von beschichteten lamellaren Substratplättchens (d.h. an die äußerste Metalloxid-haltige Schicht) oder, bei nicht ganz vollständiger Bedeckung, an die Metalloberfläche anbinden kann. Der Kohlenwasserstoffrest des Silans weist vom Pigment weg. Je nach der Art und Beschaffenheit des Kohlenwasserstoffrests des Silans wird ein unterschiedlicher Grad der Hydrophobierung des Pigments erreicht. Beispiele für solche Silane sind Hexadecyltrimethoxysilan, Propyltrimethoxysilan, etc. Besonders bevorzugt sind Pigmente auf Basis von Siliciumdioxid-beschichteten Aluminiumsubstratplättchen mit einem monofunktionellen Silan oberflächenmodifiziert. Besonders bevorzugt sind Octyltrimethoxysilan, Octyltriethoxysilan, Hecadecyltrimethoxysilan sowie Hecadecyltriethoxysilan. Durch die veränderten Oberflächeneigenschaften / Hydrophobierung kann eine Verbesserung bezüglich Haftung, Abriebfestigkeit und Ausrichtung in der Anwendung erzielt werden.

Geeignete Pigmente auf Basis eines lamellaren Substratplättchens umfassen beispielsweise die Pigmente der Reihe VISIONAIRE von Eckart.

Pigmente auf Basis eines lentikularen Substratplättchens sind beispielsweise unter der Bezeichnung Alegrace^{®} Gorgeous von der Firma Schlenk Metallic Pigments GmbH erhältlich.

Pigmente auf Basis eines Substratplättchens, welches ein Vakuum metallisiertes Pigment umfasst, sind beispielsweise unter der Bezeichnung Alegrace^{®} Marvelous oder Alegrace^{®} Aurous von der Firma Schlenk Metallic Pigments GmbH erhältlich.

Im Rahmen einer weiteren Ausführungsform ist ein erfindungsgemäßes Verfahren dadurch gekennzeichnet, dass die Zusammensetzung (A) - bezogen auf das Gesamtgewicht der Zusammensetzung (A) - ein oder mehrere Pigmente in einer Gesamgmten von 0,001 bis 20 Gew.-%, insbesondere von 0,05 bis 5 Gew.-%, enthält.

Im Rahmen einer weiteren Ausführungsform ist ein erfindungsgemäßes Verfahren dadurch gekennzeichnet, dass die Zusammensetzung (B) - bezogen auf das Gesamtgewicht der Zusammensetzung (B) - ein oder mehrere Pigmente in einer Gesamgmten von 0,001 bis 20 Gew.-%, insbesondere von 0,05 bis 5 Gew.-%, enthält.

Als farbgebende Verbindungen können die erfindungsgemäßen Mittel auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L. Besonders bevorzugt besitzen die direktziehenden Farbstoffe im Sinne der vorliegenden Erfindung eine Löslichkeit in Wasser (760 mmHg) bei 25 °C von mehr als 1,5 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung mindestens einen anionischen, kationischen und/oder nichtionischen direktziehenden Farbstoff enthält.

In einer weiteren bevorzugten Ausführungsform ist ein erfindungsgemäßes Verfahren dadurch gekennzeichnet, dass die Zusammensetzung (B) und/oder die Zusammensetzung (C) mindestens eine farbgebende Verbindung aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%. Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Weiterhin können auch thermochrome Farbstoffe eingesetzt werden. Thermochromie beinhaltet die Eigenschaft eines Materials, in Abhängigkeit der Temperatur reversibel oder irreversibel seine Farbe zu ändern. Dies kann sowohl durch Änderung der Intensität und/oder des Wellenlängenmaximums erfolgen.

Schließlich ist es auch möglich, photochrome Farbstoffe einzusetzen. Photochromie beinhaltet die Eigenschaft eines Materials, in Abhängigkeit der Bestrahlung mit Licht, insbesondere UV-Licht, reversibel oder irreversibel seine Farbe zu ändern. Dies kann sowohl durch Änderung der Intensität und/oder des Wellenlängenmaximums erfolgen.

### Anwendung der Zusammensetzungen (A) und (B)

Das erfindungsgemäße Verfahren umfasst die Anwendung der beiden Zusammensetzungen (A) und (B) auf dem keratinischen Material. Bei den beiden Zusammensetzungen (A) und (B) handelt es sich im zwei verschiedene Zusammensetzungen.

Wie bereits zuvor beschrieben ist es besonders bevorzugt, wenn zuerst die Zusammensetzung (A) auf dem Keratinmaterial appliziert wird, und im Anschluss daran die Zusammensetzung (B) in Form eines Nachbehandlungsmittel auf das Keratinmaterial aufgetragen wird.

Im Rahmen einer weiteren Ausführungsform besonders bevorzugt ist ein erfindungsgemäßes Verfahren umfassend die folgenden Schritte:
(1) Auftragen des ersten Zusammensetzung (A) auf das Keratinmaterial,
(2) Einwirkenlassen der Zusammensetzung (A) auf das Keratinmaterial für einen Zeitraum von 1 bis 10 Minuten, bevorzugt 1 bis 5 Minuten,
(3) Ausspülen der Zusammensetzung (A) aus dem Keratinmaterial,
(4) Auftragen der Zusammensetzung (B) auf das Keratinmaterial,
(5) Einwirkenlassen der Zusammensetzung (B) auf das Keratinmaterial für einen Zeitraum von 1 bis 10 Minuten, bevorzugt 1 bis 5 Minuten,
(6) Ausspülen der Zusammensetzung (B) aus dem Keratinmaterial.

Unter dem Ausspülen des keratinischen Materials mit Wasser in den Schritten (3) und (6) des Verfahrens wird erfindungsgemäß verstanden, dass für den Ausspülvorgang nur Wasser verwendet wird, ohne dass noch weitere, von den Zusammensetzungn (a) und (b) verschiedene Zusammensetzung zur Anwendung kämen.

In einem Schritt (1) wird zunächst das Zusammensetzung (A) auf die Keratinmaterialien, insbesondere die menschlichen Haare, appliziert.

Nach dem Auftragen wird das Zusammensetzung (A) auf die Keratinmaterialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des erfindungsgemäßen Verfahrens kann die Zusammensetzung (A) nun von den Keratinmaterialien ausgespült werden, bevor die Zusammensetzung (B) im nachfolgenden Schritt auf die Haare appliziert wird.

In Schritt (4) wird nun das Zusammensetzung (B) auf die Keratinmaterialien appliziert. Nach dem Auftragen wird nun das Zusammensetzung (B) auf die Haare einwirken gelassen.

Das erfindungsgemäße Verfahren erlaubt selbst bei kurzer Einwirkzeit des Zusammensetzungen (A) und (B) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In Schritt (6) wird nun das Zusammensetzung (B) mit Wasser aus dem Keratinmaterial ausgespült.

Im Rahmen einer weiteren Ausführungsform ganzbesonders bevorzugt ist ein erfindungsgemäßes Verfahren umfassend die folgenden Schritte in der angegebenen Reihenfolge:
(1) Auftragen des ersten Zusammensetzung (A) auf das Keratinmaterial,
(2) Einwirkenlassen der Zusammensetzung (A) auf das Keratinmaterial für einen Zeitraum von 1 bis 10 Minuten, bevorzugt 1 bis 5 Minuten,
(3) Ausspülen der Zusammensetzung (A) aus dem Keratinmaterial,
(4) Auftragen der Zusammensetzung (B) auf das Keratinmaterial,
(5) Einwirkenlassen der Zusammensetzung (B) auf das Keratinmaterial für einen Zeitraum von 1 bis 10 Minuten, bevorzugt 1 bis 5 Minuten,
(6) Ausspülen der Zusammensetzung (B) aus dem Keratinmaterial.

### Mehrkomponenten-Verpackungseinheit (Kit-of-parts)

Zur Erhöhung des Anwender-Komforts werden dem Anwender alle für den Anwendungsprozess, insbesondere für den Färbeprozess, notwendigen Zubereitungen in Form einer Mehrkomponenten-Verpackungseinheit (Kit-of-parts) zur Verfügung gestellt.

Ein zweiter Gegenstand der vorliegenden Erfindung ist eine Mehrkomponenten-Verpackungseinheit (Kit-of-parts) zum Behandeln von keratinischem Material, umfassend getrennt voneinander konfektioniert
- einen ersten Container mit einer ersten Zusammensetzung (A) und
- einen zweiten Container mit einer zweiten Zusammensetzung (B),
wobei die Zusammensetzungen (A) und (B) bereits im Detail bei der Beschreibung des ersten Erfindungsgegenstands offenbart wurden.

Weiterhin kann die erfindungsgemäße Mehrkomponenten-Verpackungseinheit auch noch eine dritte Verpackungseinheit enthaltend eine kosmetische Zubereitung (C) umfassen. Die Zubereitung (C) enthält wie zuvor beschrieben ganz besonders bevorzugt mindestens eine farbgebende Verbindung.

In einer ganz besonders bevorzugten Ausführungsform umfasst die erfindungsgemäße Mehrkomponenten-Verpackungseinheit (Kit-of-parts) getrennt voneinander konfektioniert
- einen dritten Container mit einer dritten Zusammensetzung (C), wobei die dritte Zusammensetzung (C) mindestens eine farbgebende Verbindung as der Gruppe der Pigmente und/oder der direktziehenden Farbstoffe enthält.

Die farbgebenden Verbindungen aus der Gruppe der Pigmente und der direktziehenden Farbstoffe sind bei der Beschreibung des ersten Erfindungsgegenstand bereits im Detail offenbart worden.

Betreffend die weiteren bevorzugten Ausführungsformen der erfindungsgemäßen Mehrkomponenten-Verpackungseinheit gilt *mutatis mutantis* das zum erfindungsgemäßen Verfahren gesagte.

## Patentansprüche

1. Verfahren zum Behandeln von keratinischem Material, insbesondere menschlichen Haaren, bei dem auf dem keratinischen Material angewendet werden:
- eine erste Zusammensetzung (A), die enthält:
(A11) mindestens ein organisches C₁-C₆-Alkoxy-Silan, das ausgewählt ist aus der Gruppe aus (3-Aminopropyl)triethoxysilan, (3-Aminopropyl)trimethoxysilan, (2-Aminoethyl)triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxy-silan, (2-Dimethylaminoethyl)trimethoxysilan und/oder deren Kondensationsprodukten, und
(A12) mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten,
- eine zweite Zusammensetzung (B), die einen pH-Wert von 8,0 bis 10,5 besitzt und die enthält (B1) mindestens eine Verbindung, die ausgewählt ist aus der Gruppe aus Aminosäuren und Proteinhydrolysaten,
wobei
- der Gesamtgehalt aller in der Zusammensetzung (A) enthaltenen Polymere - bezogen auf das Gesamtgewicht der Zusammensetzung (A) - unterhalb von 0,1 Gew.-% liegt, und
- der Gesamtgehalt aller in der Zusammensetzung (B) enthaltenen Polymere - bezogen auf das Gesamtgewicht der Zusammensetzung (B) - unterhalb von 0,1 Gew.-% liegt.

2. Verfahren nach Anspruch 1, **dadurch gekennzeichnet**, das die erste Zusammensetzung (A) mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

3. Verfahren nach einem der Ansprüche 1 bis 2, **dadurch gekennzeichnet, dass** die zweite Zusammensetzung (B) mindestens eine Aminosäure (B1) enthält, die ausgewählt ist aus der Gruppe aus Arginin, Lysin, Histidin, Asparagin, Glutamin, Cystein, Methionin, Tryptophan, Serin, Alanin, Asparaginsäure, Glutaminsäure, Glycin, Isoleucin, Leucin, Phenylalanin, Prolin, Threonin, Tyrosin und Valin.

4. Verfahren nach einem der Ansprüche 1 bis 3, **dadurch gekennzeichnet, dass** die zweite Zusammensetzung (B) - bezogen auf das Gesamtgewicht der Zusammensetzung (B) - eine oder mehrere Aminosäuren (B1) in einer Gesamtmenge von 0,1 bis 20,0 Gew.-%, bevorzugt 0,5 bis 10,0 Gew.-% enthält.

5. Verfahren nach einem der Ansprüche 1 bis 4, **dadurch gekennzeichnet, dass** die zweite Zusammensetzung (B) mindestens ein Proteinhydrolysat (B1) enthält, das ausgewählt ist aus der Gruppe aus Proteinhydrolysaten des Elastins, des Kollagens, des Keratins, der Seide, des Milcheiweißes, Soja-, Mandel-, Erbsen-, Moringa-, Kartoffel- und Weizenproteinhydrolysaten.

6. Verfahren nach einem der Ansprüche 1 bis 5, **dadurch gekennzeichnet, dass** die zweite Zusammensetzung (B) - bezogen auf das Gesamtgewicht der Zusammensetzung (B) - eine oder mehrere Proteinhydrolysate (B1) in einer Gesamtmenge von 0,1 bis 20,0 Gew.-%, bevorzugt 0,5 bis 10,0 Gew.-% enthält.

7. Verfahren nach einem der Ansprüche 1 bis 6, **dadurch gekennzeichnet, dass** der Gesamtgehalt aller in der Zusammensetzung (A) enthaltenen Polymere - bezogen auf das Gesamtgewicht der Zusammensetzung (A) - unterhalb von 0,01 Gew.-% liegt.

8. Verfahren nach einem der Ansprüche 1 bis 7, **dadurch gekennzeichnet, dass** der Gesamtgehalt aller in der Zusammensetzung (B) enthaltenen Polymere - bezogen auf das Gesamtgewicht der Zusammensetzung (B) - unterhalb von 0,01 Gew.-% liegt.

9. Verfahren nach einem der Ansprüche 1 bis 8, **dadurch gekennzeichnet, dass** die erste Zusammensetzung (A) mindestens eine farbgebende Verbindung aus der Gruppe der Pigmente und/oder der direktziehenden Farbstoffe enthält.

10. Verfahren nach einem der Ansprüche 1 bis 9, **dadurch gekennzeichnet, dass** die zweite Zusammensetzung (B) mindestens eine farbgebende Verbindung aus der Gruppe der Pigmente und/oder der direktziehenden Farbstoffe enthält.

11. Verfahren nach einem der Ansprüche 1 bis 10, umfassend die folgenden Schritte:
(1) Auftragen des ersten Mittels (A) auf das Keratinmaterial,
(2) Einwirkenlassen des Mittels (A) auf das Keratinmaterial für einen Zeitraum von 1 bis 10 Minuten, bevorzugt 1 bis 5 Minuten,
(3) Ausspülen des Mittels (A) aus dem Keratinmaterial,
(4) Auftragen des Mittels (B) auf das Keratinmaterial,
(5) Einwirkenlassen des Mittels (B) auf das Keratinmaterial für einen Zeitraum von 1 bis 10 Minuten, bevorzugt 1 bis 5 Minuten,
(6) Ausspülen des Mittel (B) aus dem Keratinmaterial.

12. Mehrkomponenten-Verpackungseinheit (Kit-of-parts) zum Behandeln von keratinischem Material, umfassend getrennt voneinander konfektioniert
- einen ersten Container mit einer ersten Zusammensetzung (A) und
- einen zweiten Container mit einer zweiten Zusammensetzung (B), wobei
die Zusammensetzungen (A) und (B) in einem der Ansprüche 1 bis 10 definiert sind.

## Claims

1. A method of treating keratinous material, in particular human hair, comprising applying to the keratinous material
- a first composition (A) comprising:
(A11) at least one organic C₁-C₆alkoxy silane selected from the group consisting of (3-aminopropyltriethoxysilane, (3-aminopropyl)trimethoxysilane, (2-aminoethyl)triethoxysilane, (2-aminoethyltrimethoxysilane, (3-dimethylaminopropyl)-triethoxysilane, (3-dimethylaminopropyltrimethoxysilane, (2-dimethylaminoethyl)-triethoxy-silane, (2-dimethylaminoethyl)trimethoxysilane and/or condensation products thereof, and
(A12) at least one organic C₁-C₆alkoxysilane selected from the group consisting of methyltrimethoxysilane, methyltriethoxysilane, ethyltrimethoxysilane, ethyltriethoxysilane, hexyltrimethoxysilane, hexyltriethoxysilane, octyltrimethoxysilane, octyltriethoxysilane, dodecyltrimethoxysilane, dodecyltriethoxysilane and/or condensation products thereof,
- a second composition (B) which has a pH of from 8.0 to 10.5 and which contains (B1) at least one compound selected from the group consisting of amino acids and protein hydrolysates,
wherein
- the total content of all polymers contained in the composition (A) - based on the total weight of the composition (A) - is below 0.1% by weight, and
- the total content of all polymers contained in the composition (B) - based on the total weight of the composition (B) - is below 0.1% by weight.

2. A method according to claim 1, **characterised in that** the first composition (A) contains at least one cosmetic ingredient from the group consisting of hexamethyldisiloxane, octamethyltrisiloxane, octamethyltrisiloxane and octamethyltrisiloxane. octamethyltrisiloxane, decamethyltetrasiloxane, hexamethylcyclotrisiloxane, octamethylcyclotetrasiloxane and decamethylcyclopentasiloxane.

3. A method according to any one of claims 1 to 2, **characterised in that** the second composition (B) comprises at least one amino acid (B1) selected from the group consisting of arginine, lysine, histidine, asparagine, glutamine, cysteine, methionine, tryptophan, serine, alanine, aspartic acid, glutamic acid, glycine, isoleucine, leucine, phenylalanine, proline, threonine, tyrosine and valine.

4. Process according to one of claims 1 to 3, **characterised in that** the second composition (B) contains - based on the total weight of the composition (B) - one or more amino acids (B1) in a total amount of 0.1 to 20.0% by weight, preferably 0.5 to 10.0% by weight.

5. Method according to one of claims 1 to 4, **characterised in that** the second composition (B) contains at least one protein hydrolysate (B1) selected from the group consisting of protein hydrolysates of elastin, collagen, keratin, silk, milk protein, soy, almond, pea, moringa, potato and wheat protein hydrolysates.

6. Method according to one of claims 1 to 5, **characterised in that** the second composition (B) contains - based on the total weight of the composition (B) - one or more protein hydrolysates (B1) in a total amount of 0.1 to 20.0% by weight, preferably 0.5 to 10.0% by weight.

7. Method according to one of claims 1 to 6, **characterised in that** the total content of all polymers contained in the composition (A) - based on the total weight of the composition (A) - is below 0.01% by weight.

8. A method according to any one of claims 1 to 7, **characterised in that** the total content of all polymers contained in the composition (B) - based on the total weight of the composition (B) - is below 0.01% by weight.

9. A method according to one of claims 1 to 8, **characterised in that** the first composition (A) contains at least one colouring compound from the group of pigments and/or direct-drawing dyes.

10. A method according to one of claims 1 to 9, **characterised in that** the second composition (B) contains at least one colouring compound from the group of pigments and/or direct-drawing dyes.

11. The method according to any one of claims 1 to 10, comprising the following steps:
(1) applying the first composition (A) to the keratin material,
(2) allowing the agent (A) to act on the keratin material for a period of 1 to 10 minutes, preferably 1 to 5 minutes,
(3) rinsing the agent (A) out of the keratin material,
(4) applying the agent (B) to the keratin material,
(5) allowing the agent (B) to act on the keratin material for a period of 1 to 10 minutes, preferably 1 to 5 minutes,
(6) rinsing the agent (B) out of the keratin material.

12. Multicomponent packaging unit (kit-of-parts) for treating keratinous material, comprising separately assembled
- a first container with a first composition (A) and
- a second container with a second composition (B), wherein
the compositions (A) and (B) are defined in one of claims 1 to 10.

## Revendications

1. Procédé de traitement de matières kératiniques, en particulier de cheveux humains, dans lequel on applique sur la matière kératinique :
- une première composition (A), qui contient
(A11) au moins un C₁-C₆-alcoxy-silane organique choisi dans le groupe constitué par le (3-aminopropyl)triéthoxysilane, le (3-aminopropyl)triméthoxysilane, le (2-aminoéthyl)triéthoxysilane, le (2-aminoéthyl)triméthoxysilane, (3-diméthylaminopropyl)-triéthoxysilane, (3-diméthylaminopropyl)triméthoxysilane, (2-diméthylaminoéthyl)-triéthoxy-silane, (2-diméthylaminoéthyl)triméthoxysilane et/ou leurs produits de condensation, et
(A12) au moins un C₁-C₆-alcoxysilane organique choisi dans le groupe constitué par le méthyltriméthoxysilane, le méthyltriéthoxysilane, l'éthyltriméthoxysilane, l'éthyltriéthoxysilane, l'hexyltriméthoxysilane, l'hexyltriéthoxysilane, l'octyltriméthoxysilane, l'octyltriéthoxysilane, le dodécyltriméthoxysilane, le dodécyltriéthoxysilane et/ou leurs produits de condensation,
- une deuxième composition (B) qui a un pH de 8,0 à 10,5 et qui contient (B1) au moins un composé choisi dans le groupe constitué par les acides aminés et les hydrolysats de protéines,
où
- la teneur totale en tous les polymères contenus dans la composition (A) - sur la base du poids total de la composition (A) - est inférieure à 0,1 % en poids, et
- la teneur totale en tous les polymères contenus dans la composition (B) - par rapport au poids total de la composition (B) - est inférieure à 0,1 % en poids.

2. Procédé selon la revendication 1, **caractérisé en ce que** la première composition (A) comprend au moins un ingrédient cosmétique choisi dans le groupe constitué par l'hexaméthyldisiloxane. Octaméthyltrisiloxane, décaméthyltétrasiloxane, hexaméthylcyclotrisiloxane, octamethyl-cyclotétrasiloxane et décaméthylcyclopentasiloxane.

3. Procédé selon l'une quelconque des revendications 1 à 2, **caractérisé en ce que** la seconde composition (B) contient au moins un acide aminé (B1) choisi dans le groupe constitué par l'arginine, la lysine, l'histidine, l'asparagine, la glutamine, la cystéine, la méthionine, le tryptophane, la sérine, l'alanine, l'acide aspartique, l'acide glutamique, la glycine, l'isoleucine, la leucine, la phénylalanine, la proline, la thréonine, la tyrosine et la valine.

4. Procédé selon l'une des revendications 1 à 3, **caractérisé en ce que** la deuxième composition (B) contient - par rapport au poids total de la composition (B) - un ou plusieurs acides aminés (B1) en une quantité totale de 0,1 à 20,0 % en poids, de préférence de 0,5 à 10,0 % en poids.

5. Procédé selon l'une quelconque des revendications 1 à 4, **caractérisé en ce que** la seconde composition (B) comprend au moins un hydrolysat de protéines (B1) choisi dans le groupe constitué par les hydrolysats de protéines d'élastine, de collagène, de kératine, de soie, de protéines de lait, de soja, d'amande, de pois, de moringa, de pomme de terre et de blé.

6. Procédé selon l'une des revendications 1 à 5, **caractérisé en ce que** la deuxième composition (B) contient - par rapport au poids total de la composition (B) - un ou plusieurs hydrolysats de protéines (B1) en une quantité totale de 0,1 à 20,0 % en poids, de préférence de 0,5 à 10,0 % en poids.

7. Procédé selon l'une quelconque des revendications 1 à 6, **caractérisé en ce que** la teneur totale en tous les polymères contenus dans la composition (A) - par rapport au poids total de la composition (A) - est inférieure à 0,01 % en poids.

8. Procédé selon l'une quelconque des revendications 1 à 7, **caractérisé en ce que** la teneur totale en tous les polymères contenus dans la composition (B) - par rapport au poids total de la composition (B) - est inférieure à 0,01 % en poids.

9. Procédé selon l'une des revendications 1 à 8, **caractérisé en ce que** la première composition (A) contient au moins un composé colorant choisi dans le groupe des pigments et/ou des colorants directs.

10. Procédé selon l'une des revendications 1 à 9, **caractérisé en ce que** la deuxième composition (B) contient au moins un composé colorant choisi dans le groupe des pigments et/ou des colorants directs.

11. Procédé selon l'une quelconque des revendications 1 à 10, comprenant les étapes suivantes :
(1) Application du premier agent (A) sur la kératine,
(2) laisser agir l'agent (A) sur la matière kératinique pendant une durée de 1 à 10 minutes, de préférence de 1 à 5 minutes,
(3) Rinçage de l'agent (A) de la matière kératinique,
(4) Application du produit (B) sur la kératine,
(5) laisser agir l'agent (B) sur la matière kératinique pendant une durée de 1 à 10 minutes, de préférence de 1 à 5 minutes,
(6) Rinçage de l'agent (B) de la matière kératinique.

12. Unité d'emballage à plusieurs composants (kit-of-parts) pour le traitement de la matière kératinique, comprenant, confectionnés séparément les uns des autres
- un premier conteneur contenant une première composition (A), et
- un deuxième conteneur contenant une deuxième composition (B), dans lequel
les compositions (A) et (B) sont définies dans l'une quelconque des revendications 1 à 10.
